# EUROPÄISCHE PATENTSCHRIFT

(11) **EP 3 662 991 B1**
(45) Veröffentlichungstag und Bekanntmachung des Hinweises auf die Patenterteilung: **25.08.2021**
(21) Anmeldenummer: 19200752.4
(22) Anmeldetag: 01.10.2019
(51) Int. Cl.: B01F 13/00, B01F 15/02

(54) **VORRICHTUNG UND VERFAHREN ZUM MISCHEN EINES KNOCHENZEMENTS MIT HOHLRAUM ZUM MONOMERTRANSFER**
DEVICE AND PROCESS FOR MIXING A BONE CEMENT WITH A CAVITY FOR MONOMER TRANSFER
DISPOSITIF ET PROCÉDÉ DE MÉLANGE D'UN CIMENT OSSEUX POURVU DE CAVITÉ POUR UN TRANSFERT AU MONOMÈRE

(30) Priorität: 07.12.2018 DE 102018131268
(43) Veröffentlichungstag der Anmeldung: 10.06.2020
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- WO-A1-2012/115022
- US-A- 3 164 303
- US-A- 5 971 953
- US-A1- 2014 254 303

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einer solchen Vorrichtung.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Es handelt sich bei der erfindungsgemäßen Vorrichtung bevorzugt um ein Full-Prepacked-Mischsystem. Besonders bevorzugt ist die Vorrichtung so ausgebildet, dass ein Austragen von gemischtem Knochenzementteig ohne die Verwendung einer separaten Auspressvorrichtung möglich ist.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt. PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2, der EP 0 796 653 A2, der US 5 588 745 A, der US 2018/333 176 A1, der US 2018/310 974 A1, der US 2018/289 406 A1, der US 2018/132 919 A1, der US 2018/132 917 A1 und der US 2018/256 233 A1 vorgeschlagen.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschließen einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung vieler bisher bekannten Full-Prepacked-Mischsysteme muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die US 3 164 303 A offenbart eine Vorrichtung zum Mischen eines Knochenzementteigs mit einem Mischer, wobei ein Kolben mit dem Mischer in zwei entgegengesetzte Richtungen bewegbar ist, um die Mischung zu ermöglichen und anschließend auszutreiben.

In der US 2018/132 917 A1 und der US 2018/132 919 A1 wurden Full-Prepacked-Mischsysteme mit einer Kartusche enthaltend ein Knochenzementpulver vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Förderkolben, mit dem der Monomerflüssigkeitsbehälter zerquetscht werden kann und die Monomerflüssigkeit aus der Aufnahme in die Kartusche gepresst werden kann. Bei diesem Mischsystem wird Monomerflüssigkeit in verdichtetes Zementpulver eingepresst, wobei das Zementpulver durch die Monomerflüssigkeit benetzt wird und die zwischen den Zementpulverpartikeln befindliche Luft durch die Monomerflüssigkeit herausgepresst wird. Das bedeutet, es entsteht ein blasenfreier Zementteig ohne Einwirkung von mechanischen Mischvorrichtungen. Für die Funktion des Mischsystems ist es zwingend notwendig, eine separate mechanische Auspressvorrichtung mit dem Kartuschensystem zu verbinden. Durch manuelle Betätigung der Auspressvorrichtung wird zuerst der Monomerflüssigkeitsbehälter geöffnet, anschließend wird die Monomerflüssigkeit in das Zementpulver gepresst, wobei der Zementteig entsteht. Anschließend wird durch weitere Betätigung der Auspressvorrichtung der gebildete Zementteig aus der Kartusche ausgepresst. Üblich sind gegenwärtig resterilisierbare Auspressvorrichtungen, die nach Gebrauch gereinigt und sterilisiert werden müssen.

Die Aufgabe der vorliegenden Erfindung besteht darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten und zum Austragen des gemischten Knochenzementteigs vorgesehen und geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer solchen Vorrichtung hergestellt wird, mit der die Nachteile der bisherigen Vorrichtungen und Verfahren überwunden werden. Die Erfindung hat die Aufgabe eine Vorrichtung wie die nach der US 2018/132 917 A1 und der US 2018/132 919 A1 derart zu verbessern, dass eine vorherbestimmbare Menge der Monomerflüssigkeit möglichst ohne Gaseinschlüsse oder Lufteinschlüsse in das Zementpulver eingepresst werden kann. Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann.

Die Vorrichtung soll möglichst ohne eine Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Aufgabe der Erfindung besteht also auch in der Entwicklung einer Vorrichtung zum Vermischen von Zementpulver und Monomerflüssigkeit. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung direkt betätigen können. Montage- und Arbeitsschritte sollen durch den Aufbau der Vorrichtung vermieden werden. In der Vorrichtung soll Zementpulver und Monomerflüssigkeit separat gelagert werden können. Bei der Vorrichtung soll es sich bevorzugt um ein Full-Prepacked-Mischsystem handeln. Eine Vermischung der beiden Zementkomponenten soll hierzu innerhalb weniger Sekunden in der geschlossenen Vorrichtung möglich sein, ohne dass ein manuelles Mischen mit Mischrädern beziehungsweise Mischflügeln notwendig ist. Die Vermischung soll dabei möglichst so erfolgen, dass der medizinische Anwender keine Berührung mit dem Zementpulver und der Monomerflüssigkeit hat. Das Mischsystem soll weiterhin so beschaffen sein, dass keine Montageschritte und auch kein externes Vakuum zum Monomertransfer notwendig sind. Das zu entwickelnde Mischsystem soll es ermöglichen, dass nach der Vermischung der beiden Zementkomponenten der gebildete Zementteig ohne Anschluss einer externen Auspressvorrichtung vorzugsweise durch manuelle Betätigung der Vorrichtung selbst ausgepresst werden kann. Die Vorrichtung soll eine Zubereitung und Applikation von Polymethylmethacrylat-Knochenzementteig ermöglichen, ohne dass Zusatzausrüstungen, wie Vakuumquellen, Vakuumschläuche und Auspressvorrichtungen erforderlich sind. Weiterhin soll der Transfer der Monomerflüssigkeit in der Weise erfolgen, dass nur Monomerflüssigkeit ohne Gaseinschlüsse beziehungsweise Luftblasen und nicht ein Gemisch aus Luft und Monomerflüssigkeit in das Zementpulver transferiert wird. Dadurch soll eine Bildung von Lufteinschlüssen im Knochenzementteig vermieden werden. Die Vorrichtung soll bevorzugt als Standsystem ausgebildet werden, so dass die Vorrichtung senkrecht auf einen Tisch gestellt werden kann und in dieser Stellung der komplette Ablauf des Transfers der Monomerflüssigkeit in das Zementpulver und das Vermischen des Zementpulvers mit der Monomerflüssigkeit sowie gegebenenfalls die Öffnung des Monomerflüssigkeitsbehälter erfolgen kann, ohne das ein Lageänderung der Vorrichtung notwendig ist.

Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des Knochenzementteigs, die Vorrichtung aufweisend
1) eine Kartusche mit einem zylindrischen Innenraum,
2) einen Kartuschenkopf mit einer Austragsöffnung zum Austreiben des Knochenzementteigs, wobei der Kartuschenkopf die Kartusche an einer Vorderseite der Kartusche bis auf die Austragsöffnung verschließt,
3) einen Förderkolben, der im Innenraum der Kartusche angeordnet ist und der im Innenraum der Kartusche in Richtung der Austragsöffnung drückbar gelagert ist,
4) einen Austragskolben, der im Innenraum der Kartusche zwischen der Austragsöffnung und dem Förderkolben angeordnet ist und der im Innenraum der Kartusche in Richtung der Austragsöffnung drückbar gelagert ist,
5) einen ersten Hohlraum, der von dem Kartuschenkopf, von Innenwänden der Kartusche und von dem Austragskolben begrenzt ist, wobei das Zementpulver in dem ersten Hohlraum angeordnet ist,
6) einen zweiten Hohlraum, der ein Teil des zylindrischen Innenraums der Kartusche ist, wobei der zweite Hohlraum von dem Austragskolben und dem Förderkolben begrenzt ist,
7) eine Fluidöffnung, die in dem Förderkolben angeordnet ist und durch die die Monomerflüssigkeit in den zweiten Hohlraum einleitbar ist,
8) eine Durchführung, die in dem Austragskolben und/oder in der Wandung der Kartusche angeordnet ist und die den ersten Hohlraum und den zweiten Hohlraum für die Monomerflüssigkeit durchlässig aber für das Zementpulver undurchlässig miteinander verbindet, und
9) ein Verschlussmittel, das an einer der Austragsöffnung abgewandten Rückseite des Austragskolbens angeordnet ist, wobei das Verschlussmittel von dem Austragskolben in Richtung des Förderkolbens absteht und von der Fluidöffnung in dem Förderkolben beabstandet ist, wobei mit dem Verschlussmittel die Fluidöffnung verschließbar ist, wenn der Förderkolben in Richtung der Austragsöffnung gedrückt wird, und wobei das Verschlussmittel derart in der mit dem Verschlussmittel verschlossenen Fluidöffnung beweglich ist, dass die Fluidöffnung verschlossen bleibt, während der Förderkolben weiter in Richtung des Austragskolbens drückbar ist.

Es kann auch vorgesehen sein, dass neben der Fluidöffnung noch zumindest eine weitere Fluidöffnung zu dem gleichen Zweck vorgesehen ist. Hierbei kann dann vorgesehen sein, dass zumindest ein zusätzliches Verschlussmittel neben dem Verschlussmittel an der Rückseite des Austragskolbens angeordnet ist, mit dem die zumindest eine weitere Fluidöffnung verschließbar ist. Die zumindest eine weitere Fluidöffnung und das zumindest eine zusätzliche Verschlussmittel können dabei, wo möglich, die im folgenden geschilderten Vorteile und Eigenschaften aufweisen, die der Fluidöffnung beziehungsweise dem Verschlussmittel zugeordnet sind.

Es sei an diese Stelle ausdrücklich darauf hingewiesen, dass der Knochenzementteig aus der Vorrichtung auf einen Spatel oder in ein Gefäß zur späteren Verwendung ausgetragen werden kann. Eine direkte Applikation an einem Patienten ist also nicht notwendig.

Bevorzugt ist die Fluidöffnung mit dem Verschlussmittel flüssigkeitsdicht verschließbar, besonders bevorzugt flüssigkeitsdicht und gasdicht, verschließbar.

Während der Förderkolben weiter in Richtung des Austragskolbens gedrückt wird und das Verschlussmittel die Fluidöffnung verschließt, kann sich der zweite Hohlraum fortwährend verkleinern.

Bevorzugt ist die erfindungsgemäße Vorrichtung auch zum Lagern des Zementpulvers vorgesehen und besonders bevorzugt auch zum Lagern der Monomerflüssigkeit vorgesehen.

Bevorzugt kann vorgesehen sein, dass an der Vorderseite der Kartusche, insbesondere am Kartuschenkopf ein Austragsrohr befestigbar ist, wobei besonders bevorzugt das Austragsrohr die Austragsöffnung begrenzt.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Es kann erfindungsgemäß vorgesehen sein, dass eine Vorderseite des Förderkolbens, die dem Austragskolben zugewandt ist, und die Rückseite des Austragskolbens zueinander flächenbündig passende Formen aufweisen, so dass die Vorderseite des Förderkolbens an der Rückseite des Austragskolbens flächenbündig anliegt, wenn der Förderkolben gegen den Austragskolben gedrückt ist, wobei vorzugsweise das Volumen des zweiten Hohlraums dadurch auf maximal 1% des Volumens des zweiten Hohlraums in einem Ausgangszustand reduzierbar ist, wobei im Ausgangszustand das zumindest eine Verschlussmittel von der zumindest einen Fluidöffnung beabstandet ist.

Hierdurch wird erreicht, dass eine in den zweiten Hohlraum eingeleitete Monomerflüssigkeit vollständig oder zu einem großen Anteil in den ersten Hohlraum gepresst werden kann, indem der Förderkolben bei bereits verschlossener Fluidöffnung in Richtung des Austragskolbens gedrückt wird.

Ferner kann vorgesehen sein, dass eine Vorderseite des Förderkolbens, die dem Austragskolben zugewandt ist, eine sich stetig in Richtung der Fluidöffnung verjüngende Oberfläche aufweist und die Rückseite des Austragskolbens eine dazu als Negativform passende sich stetig in Richtung des Verschlussmittels verjüngende Oberfläche aufweist. Hierdurch wird verhindert, dass sich in dem zweiten Hohlraum Vorsprünge oder Hinterschneidungen bilden, die bei senkrechter Aufstellung der Vorrichtung mit dem Kartuschenkopf nach unten die Bildung von Lufteinschlüssen in der in den zweiten Hohlraum eingeleiteten Monomerflüssigkeit ermöglichen oder provozieren.

Des Weiteren kann vorgesehen sein, dass eine Vorderseite des Förderkolbens, die dem Austragskolben zugewandt ist, eine Vertiefung mit trichterförmiger Oberfläche aufweist, in deren tiefsten Punkt die Fluidöffnung angeordnet ist, und die Rückseite des Austragskolbens einen vorspringende kegelförmige Oberfläche mit der gleichen Steigung wie die trichterförmige Oberfläche aufweist, wobei an der Spitze der kegelförmigen Oberfläche das Verschlussmittel angeordnet ist.

Auf diese Weise kann zum einen die Bildung das Bildung von Gas- oder Lufteinschlüssen im zweiten Hohlraum verhindert werden und zum anderen können beim Einfüllen der Monomerflüssigkeit in den zweiten Hohlraum Gas- oder Lufteinschlüsse leicht durch die Fluidöffnung entweichen.

Dabei kann vorgesehen sein, dass die trichterförmige Oberfläche an der Vorderseite des Förderkolbens einen Steigungswinkel von mindestens 45° aufweist, bevorzugt von mindestens 55°, besonders bevorzugt von mindestens 60°, und die kegelförmige Oberfläche an der Rückseite des Austragskolbens einen dazu passenden Steigungswinkel von mindestens 45° aufweist, bevorzugt von mindestens 55°, besonders bevorzugt von mindestens 60°aufweist.

Hiermit wird bei senkrechter Aufstellung der Vorrichtung mit dem Kartuschenkopf nach unten ein schnelles Entweichen von Gaseinschlüssen aus dem zweiten Hohlraum ermöglicht.

Es kann auch vorgesehen sein, dass das Verschlussmittel ein zylindrischer Stab ist, der zumindest mit einer Länge von 10 mm von Rückseite des Austragskolbens absteht.

Hiermit wird sichergestellt, dass der zweite Hohlraum zum Einpressen einer ausreichend großen Menge der Monomerflüssigkeit groß genug ist.

Es kann vorgesehen sein, dass der Austragskolben und/oder der Förderkolben gegen eine Innenwand der Kartusche abgedichtet ist oder sind, wobei die Innenwand der Kartusche den Innenraum der Kartusche begrenzt. Dabei kann bevorzugt vorgesehen sein, dass der Austragskolben und/oder der Förderkolben mit zumindest einem Dichtungsring gegen die Innenwand der Kartusche abgedichtet ist oder sind. Hierbei kann wiederum besonders bevorzugt vorgesehen sein, dass der zumindest eine Dichtungsring in zumindest einer umlaufenden Nut im Austragskolben und/oder der Förderkolben angeordnet ist oder sind.

Durch die Abdichtung wird verhindert, dass die Monomerflüssigkeit an dem Austragskolben und/oder dem Förderkolben vorbei gedrückt werden kann und so einen negativen Einfluss auf das gewünschte Mischungsverhältnis haben kann oder eine Kontamination der Umgebung verursacht.

Gemäß einer besonders bevorzugten Weiterbildung der vorliegenden Erfindung kann vorgesehen sein, dass an einer dem Austragskolben abgewandten Rückseite des Förderkolbens ein Behälter angeordnet ist, in dem ein Monomerflüssigkeitsbehälter enthaltend die Monomerflüssigkeit angeordnet ist, insbesondere eine Ampulle aus Glas oder Kunststoff, wobei der Monomerflüssigkeitsbehälter innerhalb des Behälters zu öffnen ist und wobei der Behälter über die Fluidöffnung mit dem zweiten Hohlraum flüssigkeitsdurchlässig verbunden ist, wobei bevorzugt ein Öffnungsmittel an einer dem Förderkolben gegenüberliegende Seite des Behälters angeordnet ist, mit dem der Monomerflüssigkeitsbehälter innerhalb des Behälters zu öffnen ist, wobei besonders bevorzugt das Öffnungsmittel eine an einer Kappe befestigte Hülse ist, wobei die Kappe auf ein Gewinde des Behälters schraubbar ist und die Kappe hierzu ein Gegengewinde aufweist, so dass beim Aufschrauben der Kappe der Monomerflüssigkeitsbehälter, insbesondere die Ampulle, mit der Hülse auf zumindest einen vorstehenden Stift an der Innenseite des Behälters gedrückt wird und dadurch der Monomerflüssigkeitsbehälter, insbesondere die Ampulle, aufbrechbar ist.

Hiermit wird ein Full-Prepacked-Mischsystem bereitgestellt, in dem alle Ausgangskomponenten des Knochenzementteigs, nämlich die Monomerflüssigkeit und das Zementpulver, in der Vorrichtung gelagert und gemischt werden können. Das Hantieren mit der Monomerflüssigkeit kann so innerhalb der Vorrichtung erfolgen und der Anwender ist vor dem Zementpulver und insbesondere vor der Monomerflüssigkeit geschützt.

Gemäß einer bevorzugten Ausführung der vorliegenden Erfindung liegt das Verschlussmittel auf einer Achse mit dem Behälter für den Monomerflüssigkeitsbehälter.

Bevorzugt steckt der Monomerflüssigkeitsbehälter in einer Passung in dem Behälter, so dass der Monomerflüssigkeitsbehälter in dem Behälter gehalten wird. An der Einmündung zur Fluidöffnung kann in dem Behälter ein Sieb oder ein Filter zum Zurückhalten von Splittern des Monomerflüssigkeitsbehälters angeordnet sein.

Die Hülse kann ein Rohrstück sein. Die Hülse kann auf Schultern einer Ampulle als Monomerflüssigkeitsbehälter drücken, so dass die Ampulle in dem Behälter an einem Ampullenboden der Ampulle aufgedrückt wird. Bevorzugt ist die Hülse einteilig mit der Kappe oder bildet mit der Kappe eine Einheit.

Als Monomerflüssigkeitsbehälter können vorzugsweise Glasampullen, Kunststoffampullen, Plastikampullen, Kunststoffbeutel, Folienbeutel, Kunststoffverbundbeutel und Aluminium-Kunststoffverbundbeuteln verwendet werden, die zur Lagerung der Monomerflüssigkeit geeignet sind.

Ferner kann bevorzugt auch vorgesehen sein, dass in der Wandung des Behälters oder im Öffnungsmittel zumindest eine Belüftungsöffnung angeordnet ist, die den Innenraum des Behälters mit der Umgebung verbindet.

Hierdurch kann der Innenraum des Behälters auf einfach Weise evakuiert und mit einem sterilisierenden Gas sterilisiert werden.

Gemäß einer bevorzugten Weiterbildung der vorliegenden Erfindung kann auch vorgesehen sein, dass die zumindest eine Belüftungsöffnung derart dicht im Bereich einer Schraubkappe, insbesondere einer Schraubkappe als Öffnungsmittel zum Öffnen eines Monomerflüssigkeitsbehälters, in dem Behälter angeordnet ist, so dass die zumindest eine Belüftungsöffnung durch eine Bewegung der Schraubkappe in Richtung der Kartusche verschlossen wird, bevor ein in dem Behälter angeordneter Monomerflüssigkeitsbehälter, in dem die Monomerflüssigkeit enthalten ist, durch die Bewegung der Schraubkappe geöffnet ist.

Hierdurch kann die Monomerflüssigkeit nicht aus dem Innenraum des Behälters austreten, wenn die zumindest eine Belüftungsöffnung von der sich in Richtung der Kartusche bewegenden Schraubkappe verschlossen wird, bevor der Monomerflüssigkeitsbehälter durch die Bewegung der Schraubkappe geöffnet wird, also beispielsweise im Innenraum des Behälters zerdrückt, zersplittert, aufgestochen oder aufgerissen wird.

Bei erfindungsgemäßen Vorrichtungen mit Behälter kann vorgesehen sein, dass der Behälter ein Außengewinde aufweist, das in ein Innengewinde an einem dem Kartuschenkopf gegenüberliegenden Ende der Kartusche schraubbar ist, wobei durch Einschrauben des Behälters in die Kartusche der Förderkolben in Richtung der Austragsöffnung drückbar ist und der Austragskolben mit dem Förderkolben in Richtung der Austragsöffnung drückbar ist, wobei bevorzugt das Innengewinde Teil einer Ringhülse ist, die an dem dem Kartuschenkopf gegenüberliegenden Ende der Kartusche mit der Kartusche verbunden ist.

Hierdurch kann die Vorrichtung durch eine Schraubbewegung von außen bedient werden. Die Schraubbewegung hat den Vorteil, dass ein kraftvoller Antrieb des Förderkolbens und des Austragskolbens ermöglicht wird, so dass auch zähe Knochenzementteige mit der Vorrichtung aus der Kartusche ausgepresst werden können.

Es kann auch vorgesehen sein, dass das Verschlussmittel durch die Fluidöffnung in den Behälter drückbar ist. Hierdurch kann das Verschlussmittel eine größere Länge haben, um den zweiten Hohlraum frühzeitig bezüglich der Fluidöffnung zu verschließen, so dass eine ausreichend große Menge der Monomerflüssigkeit aus dem zweiten Hohlraum in den ersten Hohlraum drückbar ist, um die gewünschte Konsistenz des Knochenzementteigs zu erhalten.

Bevorzugt kann ferner vorgesehen sein, dass die Länge des Verschlussmittels so lang ist, dass das Volumen des zweiten Hohlraums höchstens so groß ist wie das Volumen der durch die Fluidöffnung eingeleiteten Monomerflüssigkeit, insbesondere der in dem Monomerflüssigkeitsbehälter enthaltenen Monomerflüssigkeit, wenn die Spitze des Verschlussmittels auf die Fluidöffnung trifft und diese verschließt.

Hierdurch wird ermöglicht, dass Gase aus dem zweiten Hohlraum austreten können so dass nur die Monomerflüssigkeit in dem zweiten Hohlraum enthalten ist, wenn das Verschlussmittel die Fluidöffnung verschließt.

Des Weiteren kann vorgesehen sein, dass das Verschlussmittel eine zylindrische Form hat und die Fluidöffnung eine dazu passende Form hat, wobei bevorzugt das Verschlussmittel eine zylindrische Form mit kreisförmiger Grundfläche hat und die Fluidöffnung ein kreisrundes oder zylindrisches Loch ist.

Hierdurch wird sichergestellt, dass das Verschlussmittel die Fluidöffnung in jeder Stellung zuverlässig verschließen kann.

Ferner kann auch vorgesehen sein, dass ein Stopfen in der Austragsöffnung angeordnet ist, der die Austragsöffnung für das Zementpulver undurchlässig verschließt, insbesondere für Gase durchlässig verschließt, wobei vorzugsweise der Stopfen in der Austragsöffnung beweglich angeordnet ist, so dass der Stopfen durch einen Druck auf den fertig gemischten Knochenzementteig aus der Austragsöffnung heraus gedrückt werden kann, wobei besonders bevorzugt ein von außen sichtbares Markierungsmittel an dem Stopfen befestigt ist, an dessen Position von außen ablesbar ist, ob der Stopfen in der Austragsöffnung nach außen gedrückt ist.

Hiermit kann ein vorzeitiges Austreten des Knochenzementteigs verhindert werden. Durch die bevorzugte Ausgestaltung wird erreicht, dass von außen erkennbar ist, wenn der Knochenzementteig bis zur Austragsöffnung fertig gemischt ist. Dies ergibt daraus, dass der Knochenzementteig fließfähig ist, während das Zementpulver nicht fließfähig ist, so dass der Stopfen nur dann bewegt wird, wenn der Knochenzementteig fertig gemischt ist oder das Zementpulver zumindest vollständig benetzt ist und der Austragskolben über den Knochenzementteig ein Druck auf den Stopfen vermittelt.

Der Stopfen kann Teil eines Verschlusssystems sein, mit dem die Austragsöffnung verschlossen ist. Dabei kann der Stopfen in einer zylindrischen Bohrung des Verschlusssystems in axialer Richtung beweglich angeordnet sein. Das Verschlusssystem kann mit einem Außengewinde in ein Innengwinde an einem Stutzen des Kartuschenkopfs eingeschraubt sein. Selbstverständlich kann das Verschlusssystem alternativ auch eine Kappe mit einem Innengewinde aufweisen, das auf einen Stutzen am Kartuschenkopf mit einem Außengewinde geschraubt ist. Der Stutzen kann nach Entfernen des Verschlusssystems mit einem Austragsrohr verbunden werden.

Das Markierungsmittel ist bevorzugt ein Farbring.

Es kann auch vorgesehen sein, dass an dem Kartuschenkopf ein lösbar befestigter Standfuß angeordnet ist, wobei der Standfuß vorzugsweise zumindest teilweise transparent ist, wobei besonders bevorzugt ein dem Kartuschenkopf zugewandter Teil des Standfußes intransparent ist und ein von dem Kartuschenkopf abgewandtes Standfußteil transparent ist.

Hiermit kann die Vorrichtung in der gewünschten Stellung, nämlich mit dem Kartuschenkopf nach unten, bequem aufgestellt werden.

Das Markierungsmittel ist bevorzugt in dem Standfuß angeordnet. Dabei ist bevorzugt das Markierungsmittel bei noch nicht vollständig erfolgter Vermischung der Monomerflüssigkeit mit dem Zementpulver im nicht transparenten Teil des Standfußes angeordnet ist. Durch einen Druck auf den fertig gemischten Knochenzementteig kann das Markierungsmittel in den transparenten Standfußteil gedrückt werden, so dass durch den Standfuß von außerhalb der Vorrichtung optisch erkennbar ist, wenn der Knochenzementteig einsatzbereit ist.

Die Kartusche ist vorzugsweise hohlzylinderförmig. Der Kartuschenkopf kann konisch geformt sein. Dadurch verbleibt ein Totvolumen innerhalb des Kartuschenkopfs, wenn der Austragskolben bis zum Kartuschenkopf gedrückt ist. An dieser Stelle können weniger gut gemischte Anteile des Knochenzementteigs zurückgehalten werden, die nicht ausgetragen werden können.

Die Durchführung ist vorzugsweise an der Spitze oder im Kegelmantel eines Kegels an der Rückseite des Austragskolbens angeordnet.

Das Verschlussmittel ist vorzugsweise zylindrisch geformt, wobei der Außendurchmesser des Verschlussmittels genauso groß ist oder größer ist als der Innendurchmesser der Fluidöffnung. Dadurch kann die Fluidöffnung zuverlässig von dem Verschlussmittel verschlossen werden, unabhängig davon, wie tief das Verschlussmittel in die Fluidöffnung eingeschoben ist.

Es kann auch vorgesehen sein, dass das Volumen der verwendeten Monomerflüssigkeit, insbesondere das Volumen der in dem Monomerflüssigkeitsbehälter enthaltenen Monomerflüssigkeit, zumindest genauso groß ist, wie das Volumen des zweiten Hohlraums.

Gemäß einer bevorzugten Ausführung der vorliegenden Erfindung kann vorgesehen sein, dass das Zementpulver so in dem ersten Hohlraum verdichtet ist, dass die Zementpulverpartikel nicht frei beweglich sind. Hierdurch wird sichergestellt, dass sich die Monomerflüssigkeit aufgrund von Kapillarkräften schnell und gleichmäßig in dem Zementpulver ausbreiten kann.

Es kann vorgesehen sein, dass ein lösbares Sicherungsmittel mit dem Förderkolben verbunden ist, das eine Bewegung des Förderkolbens gegen die Kartusche verhindert. Bevorzugt ist das Sicherungsmittel an dem Behälter für den Monomerflüssigkeitsbehälter angeordnet und blockiert ein Einschieben oder Einschrauben des Behälters in die Kartusche und damit eine Bewegung des Förderkolbens in der Kartusche.

Die Kartusche ist vorzugsweise aus einem thermoplastischen Kunststoff gefertigt, insbesondere mit einem Spritzgussverfahren. Besonders bevorzugt sind auch die anderen Teile der Vorrichtung, wie der Austragskolben, der Förderkolben und der Kartuschenkopf, sowie gegebenenfalls der Behälter, der Stopfen und der Standfuß aus einem thermoplastischen Kunststoff gefertigt, insbesondere mit einem Spritzgussverfahren.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer zuvor beschriebenen erfindungsgemäßen Vorrichtung hergestellt wird, gekennzeichnet durch die folgenden nacheinander ablaufenden Schritte:
A) Einleiten der Monomerflüssigkeit durch die Fluidöffnung in den zweiten Hohlraum,
B) Drücken des Förderkolbens in Richtung des Austragskolbens bis das Verschlussmittel an der Rückseite des Austragskolbens die Fluidöffnung verschließt,
C) Weiteres Drücken des Förderkolbens in Richtung des Austragskolbens, wobei die Monomerflüssigkeit aus dem zweiten Hohlraum in das Zementpulver in den ersten Hohlraum gedrückt wird, das Verschlussmittel durch die Fluidöffnung oder tiefer in die Fluidöffnung geschoben wird und die Fluidöffnung verschlossen bleibt,
D) Drücken des Austragskolbens mit dem Förderkolben in Richtung der Austragsöffnung, wobei der in dem ersten Hohlraum gebildete Knochenzementteig durch die Austragsöffnung ausfließt.

Bevorzugt kann vorgesehen sein, dass bei dem erfindungsgemäßen Verfahren keine Behandlung eines menschlichen oder tierischen Körpers erfolgt, insbesondere keine von der Patentierung ausgeschlossene Behandlung eines menschlichen oder tierischen Körpers erfolgt.

Bevorzug kann ferner vorgesehen sein, dass sich der Knochenzementteig während Schritt C) und/oder nach Schritt C) aber vor Schritt D) in dem ersten Hohlraum bildet.

Es kann auch vorgesehen sein, dass die Vorrichtung in Schritt A), und vorzugsweise auch in Schritt B) und C), mit dem Kartuschenkopf nach unten aufgestellt oder gehalten wird und Gaseinschlüsse aus dem zweiten Hohlraum durch die Fluidöffnung entweichen.

Hierdurch kann eine genau vorbestimmte Menge der Monomerflüssigkeit in das Zementpulver eingetragen werden. Zudem kann so ein blasenfreier oder blasenarmer Knochenzementteig erzeugt werden.

Es ist dabei auch nicht schlimm, wenn eine geringe Menge der Lufteinschlüsse in dem zweiten Hohlraum verbleiben, insbesondere dann nicht, wenn diese in der Durchführung zwischen dem ersten Hohlraum und dem zweiten Hohlraum verbleiben und nicht in den ersten Hohlraum gelangen.

Ferner kann vorgesehen sein, dass in Schritt D) der Förderkolben flächenbündig an dem Austragskolben anliegt und bevorzugt in Schritt C) das Volumen des zweiten Hohlraums vollständig oder bis auf maximal 1% reduziert wird.

Dadurch kann die Monomerflüssigkeit vollständig aus dem zweiten Hohlraum in das Zementpulver in dem zweiten Hohlraum gepresst werden.

Des Weiteren kann vorgesehen sein, dass in Schritt D) durch den auf den Knochenzementteig wirkenden Druck ein Stopfen in der Austragsöffnung bewegt oder hervorgedrückt wird, wobei bevorzugt hierauf der Stopfen aus der Austragsöffnung entfernt wird und gegebenenfalls ein Standfuß von dem Kartuschenkopf entfernt wird und besonders bevorzugt danach ein Applikationsrohr an dem Kartuschenkopf der Kartusche befestigt wird.

Hierdurch kann ein Anwender der Vorrichtung erkennen, wann der Knochenzementteig einsatzbereit ist.

Es kann auch vorgesehen sein, dass vor Schritt A) ein Monomerflüssigkeitsbehälter enthaltend die Monomerflüssigkeit in einem Behälter geöffnet wird und die Monomerflüssigkeit in dem Behälter freigesetzt wird, wobei der Behälter an einer dem Austragskolben abgewandten Rückseite des Förderkolbens angeordnet ist, und die Monomerflüssigkeit in Schritt A) aus dem Behälter durch die Fluidöffnung in den zweiten Hohlraum fließt, wobei vorzugsweise in Schritt B) und C) der Förderkolben und in Schritt D) der Förderkolben und der Austragskolben durch Eindrücken oder Einschrauben des Behälters in die Kartusche angetrieben werden.

Hierdurch kann das Verfahren auf einfache Weise auch manuell durchgeführt werden. Die Vorrichtung kann als Full-Prepacked-Mischsystem verwendet werden und ein Kontakt des Anwenders mit der Monomerflüssigkeit kann während des Verfahrens ausgeschlossen werden.

Der Erfindung liegt die überraschende Erkenntnis zugrunde, dass es durch eine von einem Austragskolben aus verschließbare Fluidöffnung in einem Förderkolben gelingt, einen Hohlraum (vorliegend den zweiten Hohlraum) nach außen durch eine Bewegung des Förderkolbens relativ zum Austragskolben hin zu verschließen und dadurch ein nach außen geschlossenes aber in Richtung eines (ersten) Hohlraums mit dem Zementpulver darin für die Monomerflüssigkeit durchlässig verbundenes Monomerflüssigkeitsreservoir bereitzustellen, das durch eine weitere Bewegung des Förderkolbens zum Austragskolben hin in das Zementpulver drückbar ist, wobei dabei der zweite Hohlraum immer weiter verkleinert wird und wobei das Monomerflüssigkeitsreservoir bei geeigneter Aufstellung der Vorrichtung frei oder im Wesentlichen frei von Gas- beziehungsweise Lufteinschlüssen ist. Um die in dem zweiten Hohlraum enthaltene Monomerflüssigkeit in das Zementpulver zu drücken und um den fertigen Knochenzementteig auszutreiben, reicht es aus, den Förderkolben in Richtung der Austragsöffnung zu drücken. Dabei verschließt sich zunächst die Fluidöffnung, anschließend wird die Monomerflüssigkeit in den ersten Hohlraum mit dem Zementpulver transferiert und danach kann der gebildete Knochenzementteig durch gemeinsames Vortreiben des Förderkolbens mit dem Austragskolben aus der Kartusche ausgetrieben werden.

Bevorzugt kann zuvor die Monomerflüssigkeit aus einem an die Rückseite des Förderkolbens angeschlossenen und mit der Fluidöffnung verbundenen Behälter in den zweiten Hohlraum durch die Fluidöffnung geleitet werden. Der Förderkolben kann dabei durch Einschieben und/oder Einschrauben des Behälters in die Kartusche im Innenraum der Kartusche in Richtung der Austragsöffnung gedrückt werden. Besonders bevorzugt kann zuvor ein Monomerflüssigkeitsbehälter innerhalb des Behälters geöffnet werden, um die Monomerflüssigkeit innerhalb des Behälters freizusetzen. Diese besonders bevorzugte erfindungsgemäße Vorrichtung hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Es handelt sich dann um ein Full-Prepacked-Mischsystem. Der medizinische Anwender hat keinerlei Kontakt mit den einzelnen Ausgangskomponenten des Knochenzementteigs. Die Geruchsbelästigung ist dadurch nur noch minimal.

Ein besonderer Vorteil der Vorrichtung besteht auch darin, dass durch das einfache Vorwärtsbewegen des Förderkolbens bei geschlossener Fluidöffnung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Voraussetzung dafür ist die Verwendung eines Zementpulvers, das so eingestellt ist, dass es sehr gut von der Monomerflüssigkeit benetzt wird und diese durch Kapillarwirkung einsaugen kann. Die Bedienung der Vorrichtung ist maximal vereinfacht.

Die Vorteile erfindungsgemäßer Vorrichtungen und Verfahren beruhen grundsätzlich darauf, dass die an sich bekannte lineare Bewegungen so genutzt werden, um den Monomertransfer durchzuführen und um den zweiten Hohlraum nach außen zu verschließen.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte und bevorzugte erfindungsgemäße Vorrichtung zum Lagern, Vermischen und Austragen von Polymethylmethacrylat-Knochenzement kann aufweisen:
a) eine hohlzylinderförmige Kartusche,
b) einen Kartuschenkopf, der die Kartusche verschließt ist, wobei der Kartuschenkopf eine Auslassöffnung aufweist, die den Innenraum der Kartusche mit der Umgebung verbindet,
c) einen für Gase durchlässigen und für Pulverpartikel undurchlässigen Stopfen als Kartuschenverschluss, der lösbar in der Auslassöffnung des Kartuschenkopfs angeordnet ist, wobei der Kartuschenverschluss mit einem Standfuß verbunden ist,
d) einen Austragskolben, der im Innenraum der Kartusche axial beweglich angeordnet ist, wobei zumindest ein Teil der Rückseite des Austragskolbens als Kegel ausgebildet ist, wobei am oder neben dem Kegelmantel mindestens eine Öffnung als Durchführung ausgebildet ist, die für Pulverpartikel undurchlässig und für Gase und Flüssigkeiten durchlässig ist, und die Vorderseite und die Rückseite des Austragskolbens gas- und flüssigkeitsdurchlässig verbindet,
e) einen hohlzylinderförmigen Ampullenhalter als Behälter, der zumindest teilweise im Innenraum der Kartusche angeordnet ist und der axial in der Kartusche beweglich ist, wobei der Ampullenhalter an seiner dem Kartuschenkopf zugewandten Stirnseite als Förderkolben in der Form eines Hohlkegels (trichterförmig) ausgebildet ist,
f) eine Fluidöffnung in der Spitze des Hohlkegels des Ampullenhalters,
g) ein Öffnungsmittel für einen Monomerflüssigkeitsbehälter, das in der dem Kartuschenkopf abgewandten Seite des Ampullenhalters angeordnet ist,
h) einen Monomerflüssigkeitsbehälter, der im Ampullenhalter angrenzend zum Öffnungsmittel angeordnet ist,
i) einen Verschlusszylinder als Verschlussmittel, der auf der Kegelspitze des Kegels des Austragskolbens axial angeordnet ist, wobei der Verschlusszylinder einen Außendurchmesser gleich oder größer der Öffnung des Hohlkegels des Ampullenhalters hat und auf einer Achse mit der Öffnung des Ampullenhalters liegt,
j) Zementpulver, das in einem ersten Hohlraum der Kartusche angeordnet ist, der vom Kartuschenkopf mit dem Kartuschenverschluss, der Innenwand der Kartusche und dem Austragskolben begrenzt ist,
k) einen zweiten Hohlraum, der durch die Innenwand der Kartusche, der Rückseite des Austragskolbens und dem Hohlkegel des Förderkolbens begrenzt ist,
l) eine im Ampullenhalter axial verschiebbaren Hülse, die oberhalb des Monomerflüssigkeitsbehälters angeordnet ist und die gegen den Monomerflüssigkeitsbehälter axial verschiebbar ist,
m) ein Außengewinde auf der Außenseite des Ampullenhalters,
n) ein manuell zu betätigender Verschlusskopf des Ampullenhalters, der ein Innengewinde aufweist, wobei das Innengewinde des Verschlusskopfs in das Außengewinde des Ampullenhalters greift, und wobei der Verschlusskopf auf der axial verschiebbaren Hülse aufliegt,
o) ein Innengewinde am einem dem Ampullenkopf gegenüberliegenden Ende der Kartusche, das mit dem Außengewinde des Ampullenhalters im Eingriff steht, wobei
p) bei axialer Bewegung des Ampullenhalters in Richtung des Kartuschenkopfs, der Verschlusszylinder in die Fluidöffnung des Hohlkegels eintritt und die Fluidöffnung gas- und flüssigkeitsdicht verschließt und wobei bei weiterer Bewegung des Ampullenhalters in Richtung des Kartuschenkopfs das Volumen des zweiten Hohlraums vermindert wird.

Die erfindungsgemäße Vorrichtung ist vorteilhaft vom Anwender zu nutzen, weil der Anwender nach Aufstellen der Vorrichtung auf eine ebene Unterlage nur den Verschlusskopf des Ampullenhalters drehen muss und nach wenigen Sekunden einen fertig gemischten Knochenzementteig erhält, der keine oder praktisch keine Lufteinschlüsse enthält. Der Prozess des Öffnens des Monomerflüssigkeitsbehälters, des Monomertransfers und der Vermischung laufen geordnet nacheinander nur durch einfache wiederholte Drehung des Verschlusskopfs des Ampullenhalters mit dem verbundenen Ampullenhalter automatisch ab. Es sind dadurch die aus der bisherigen Zementiertechnik bekannten Anwenderfehler beim Öffnen der Monomerflüssigkeitsbehälter, dem Monomertransfer und dem Vermischen der Monomerflüssigkeit mit dem Zementpulver konstruktiv ausgeschlossen. Dadurch wird die Anwendersicherheit deutlich erhöht. Die Vorrichtung ist dabei auch ohne weiteres von ungelernten Hilfskräften einsetzbar.

Ein erfindungsgemäßes Verfahren kann beispielsweise mit der beispielhaften Vorrichtung zur Vermischung des Zementpulvers mit der Monomerflüssigkeit unter Bildung von Knochenzementteig umgesetzt werden. Ein beispielhaftes und erfindungsgemäß bevorzugtes Verfahren kann mit den folgenden und nacheinander ablaufenden Schritten umgesetzt werden:
Aufstellen der Kartusche mit dem Standfuß nach unten auf einer Unterlage,
Drehen des Verschlusskopfs auf das Außengewinde des Ampullenhalters,
Verschieben der Hülse gegen den Monomerflüssigkeitsbehälter,
Bewegen des Monomerflüssigkeitsbehälters mit der Öffnungsvorrichtung,
Öffnen des Monomerflüssigkeitsbehälters,
Ausfließen der Monomerflüssigkeit aus dem Monomerflüssigkeitsbehälter durch die Fluidöffnung des Ampullenhalters in den zweiten Hohlraum,
Verschieben des Ampullenhalters in Richtung des Kartuschenkopfs, bis das Außengewinde des Ampullenhalters in das Innengewinde der Kartusche greift,
Drehen des Verschlusskopfs mit dem verbundenen Ampullenhalter, wobei das Außengewinde des Ampullenhalters sich in das Innengewinde der Kartusche dreht und der Ampullenhalter in Richtung Kartuschenkopf bewegt wird,
Austritt der über der Monomerflüssigkeit im zweiten Hohlraum stehenden Luft oder anderer Gase durch die Fluidöffnung des Hohlkegels des Ampullenhalters,
Verschließen der Fluidöffnung des Ampullenhalters bei weiterer Bewegung des Ampullenhalters in Richtung des Kartuschenkopfs,
Auspressen der Monomerflüssigkeit aus dem zweiten Hohlraum durch die Durchführung im Austragskolben in das verdichtete Zementpulver durch Verschiebung des Ampullenhalters gegen den Austragskolben, wobei der Hohlkegel des Förderkolbens auf den Kegel des Austragskolbens auffährt und das Volumen des zweiten Hohlraums vermindert, bis der zweite Hohlraum nicht mehr oder fast nicht mehr vorhanden ist,

Bildung des Knochenzementteigs durch Anquellen des mit Monomerflüssigkeit benetzten Zementpulvers,
Verschiebung des axial beweglichen Stopfens in der Austragsöffnung durch den Knochenzementteig bei weiterer axialer Bewegung des Ampullenhalters und des Austragskolbens in Richtung des Kartuschenkopfs,
Entfernung des Kartuschenverschlusses und des Standfußes, und
bevorzugt Auspressen des Knochenzementteigs durch weiteres Drehen des Ampullenhalters.

Weiterhin ist ein zweites Verfahren erfindungsgemäß bevorzugt. Dieses Verfahren zum Vermischen und Austragen von Polymethylmethacrylat-Knochenzement mit der beispielhaften erfindungsgenmäßen Vorrichtung ist durch folgende nacheinander ablaufende Schritte charakterisiert:
Aufstellen der Kartusche mit dem Kartuschenkopf als Standfuß auf einer Unterlage,
Drehen des Verschlusskopfes auf das Außengewinde des Ampullenhalters,
Verschieben der Hülse gegen den Monomerflüssigkeitsbehälter,
Bewegen des Monomerflüssigkeitsbehälters mit der Öffnungsvorrichtung,
Öffnen des Monomerflüssigkeitsbehälters,
Ausfließen der Monomerflüssigkeit aus dem Monomerflüssigkeitsbehälters durch die Fluidöffnung des Ampullenhalters in den zweiten Hohlraum,
Drehen des Verschlusskopfes mit dem verbundenen Ampullenhalter, wobei das Außengewinde des Ampullenhalters in das Innengewinde der Kartusche greift und der Ampullenhalter in Richtung Kartuschenkopf bewegt wird,
Austritt der über der Monomerflüssigkeit im zweiten Hohlraum stehenden Luft oder eines Gases durch die Fluidöffnung des Hohlkegels des Ampullenhalters,
Verschließen der Fluidöffnung des Ampullenhalters bei weiterer Bewegung des Ampullenhalters in Richtung des Kartuschenkopfs,
Auspressen der Monomerflüssigkeit aus dem zweiten Hohlraum durch die Durchführung im Austragskolben in das verdichtete Zementpulver durch Verschiebung des Ampullenhalters gegen den Austragskolben, wobei der Hohlkegel des Ampullenhalters auf den Kegel des Austragskolbens auffährt und das Volumen des zweiten Hohlraums vermindert, bis der zweite Hohlraum nicht mehr oder im Wesentlichen nicht mehr vorhanden ist,
Bildung des Knochenzementteigs durch Anquellen des mit Monomerflüssigkeit benetzten Zementpulvers,
Verschiebung des axial beweglichen Stopfens im Kartuschenverschluss durch den Knochenzementteig bei weiterer axialer Bewegung des Ampullenhalters und des Austragskolbens in Richtung des Kartuschenkopfs,
Entfernung des Kartuschenverschlusses und des Standfußes, und
bevorzugt Auspressen des Knochenzementteigs durch weiteres Drehen es Ampullenhalters.

Eine Variante beider beispielhafter Verfahren ist durch folgende nacheinander ablaufende Schritte charakterisiert, dass beim Verschieben des beweglichen Stopfens ein mit dem beweglichen Stopfen verbundener Farbring als Markierungsmittel vor ein transparentes Fenster des Standfußes tritt und dadurch die erfolgte Vermischung des Zementpulvers mit der Monomerflüssigkeit dem medizinischen Anwender anzeigt.

Im Folgenden werden weitere Ausführungsbeispiele der Erfindung anhand von elf schematisch dargestellten Figuren erläutert, ohne jedoch dabei die Erfindung zu beschränken. Dabei zeigt:
Figur 1: eine schematische Querschnittansicht einer ersten beispielhaften erfindungsgemäßen Vorrichtung zum Lagern und Mischen einer Monomerflüssigkeit und eines Zementpulvers in einem Ausgangs- beziehungsweise Lagerungszustand der Vorrichtung;
Figur 2: eine schematische perspektivische Querschnittansicht der Vorrichtung nach Figur 1 ohne die Monomerflüssigkeit und dass Zementpulver;
Figur 3: eine schematische perspektivische Außenansicht der Vorrichtung nach den Figuren 1 und 2;
Figur 4: eine schematische Querschnittansicht der Vorrichtung nach den Figuren 1 bis 3 mit einem geöffnetem Monomerflüssigkeitsbehälter darin;
Figur 5: eine schematische Querschnittansicht der Vorrichtung nach den Figuren 1 bis 4 mit einem abgezogenem Sicherungselement;
Figur 6: eine schematische Querschnittansicht der Vorrichtung nach den Figuren 1 bis 5 bei der das Außengewinde des Behälters in das Innengewinde der mit der Kartusche verbundenen Ringhülse greift;
Figur 7: eine schematische Querschnittansicht der Vorrichtung nach den Figuren 1 bis 6 nach dem Einpressen der Monomerflüssigkeit in das Zementpulver;
Figur 8: eine schematische Querschnittansicht der Vorrichtung nach den Figuren 1 bis 7 mit vorgeschobenem Stopfen und Markierungsmittel als Anzeige zur Einsatzbereitschaft des Knochenzementteigs;
Figur 9: eine schematische Querschnittansicht der Vorrichtung nach den Figuren 1 bis 8 beim Austragen des Knochenzementteigs;
Figur 10: eine schematische Querschnittansicht einer Ausschnittvergrößerung der Vorrichtung nach den Figuren 1 bis 9 kurz vor dem Verschließen der Fluidöffnung; und
Figur 11: eine schematische Querschnittansicht einer Ausschnittvergrößerung einer alternativen zweiten erfindungsgemäßen Vorrichtung mit einem variabel einstellbaren Verschlussmittel.

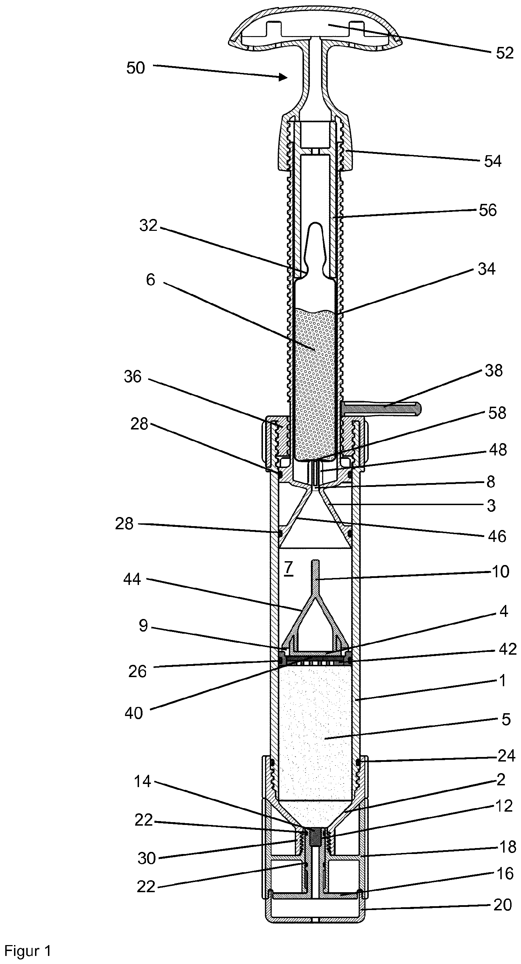

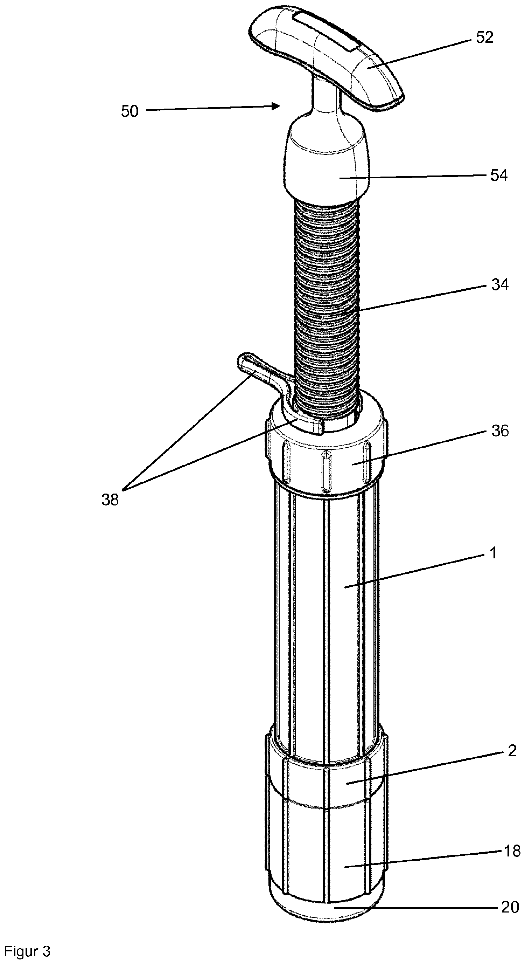

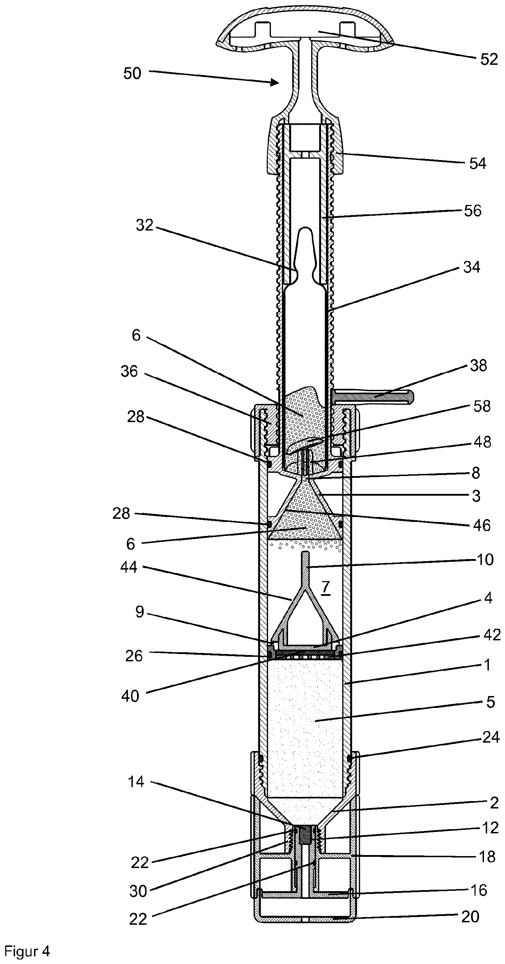

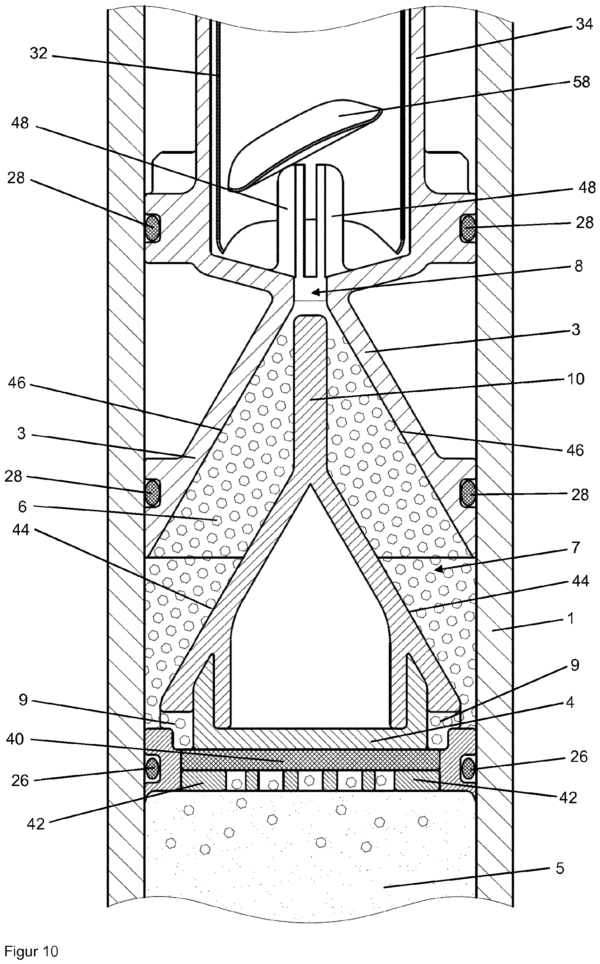

In den Figuren 1 bis 10 sind Abbildungen einer ersten erfindungsgemäßen Vorrichtung gezeigt. Die Figuren 1 bis 9 zeigen verschiedene schematische Gesamtansichten der beispielhaften ersten erfindungsgemäßen Vorrichtung. Die Figur 10 zeigt schematische Querschnittansichten als Detailansichten in Form von Ausschnittvergrößerungen durch einen Bereich der erfindungsgemäßen Vorrichtung. Die Figuren 1 bis 3 zeigen dabei die erste erfindungsgemäße Vorrichtung in einem Ausgangszustand, während die Figuren 4 bis 10 Querschnittansichten der ersten erfindungsgemäßen Vorrichtung während der Benutzung der Vorrichtung zur Verdeutlichung eines beispielhaften erfindungsgemäßen Verfahrens zeigen.

Gemäß einer bevorzugten Ausführung der vorliegenden Erfindung hat die Vorrichtung eine röhrenförmigen Kartusche 1 aus einem Kunststoff mit einem zylindrischen Innenraum. Die Kartusche 1 kann an ihrer Vorderseite mit einem trichterförmigen Kartuschenkopf 2 aus Kunststoff verschlossen sein. Die Vorderseite der Kartusche 1 ist in den Figuren 1 bis 9 unten. An der Spitze des trichterförmigen Kartuschenkopfs 2 kann eine zentrale Austragsöffnung angeordnet sein, die im Ausgangszustand aber zunächst verschlossen sein kann. Der Kartuschenkopf 2 kann gemäß einer alternativen Ausführung aber auch eine flache Kappe sein oder eine andere Form aufweisen. Es ist grundsätzlich auch möglich, den Kartuschenkopf 2 einteilig mit der Kartusche 1 auszuführen. Um die Montage der Vorrichtung zu erleichtern, wird jedoch bevorzugt, wenn der Kartuschenkopf 2 als separates Teil mit der Kartusche 1 verbunden wird, beispielsweise aufgeschraubt oder aufgesteckt wird.

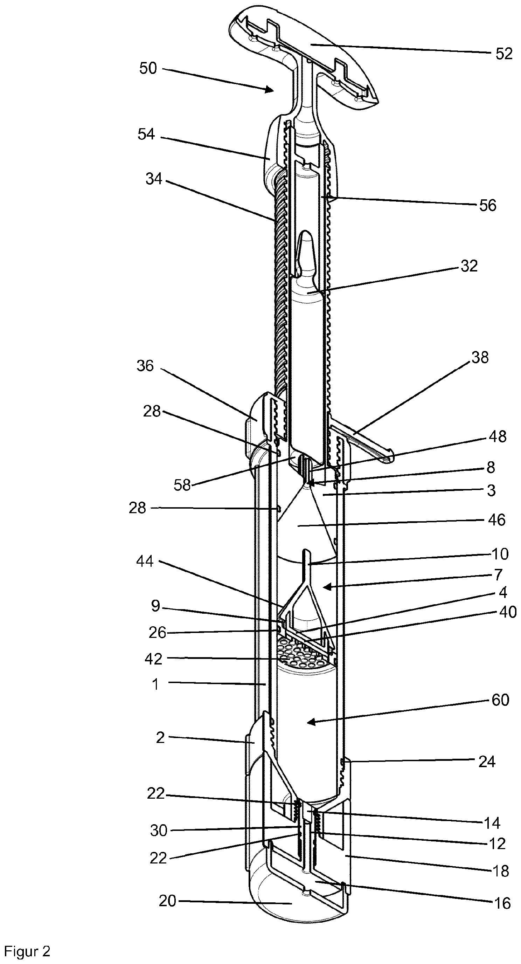

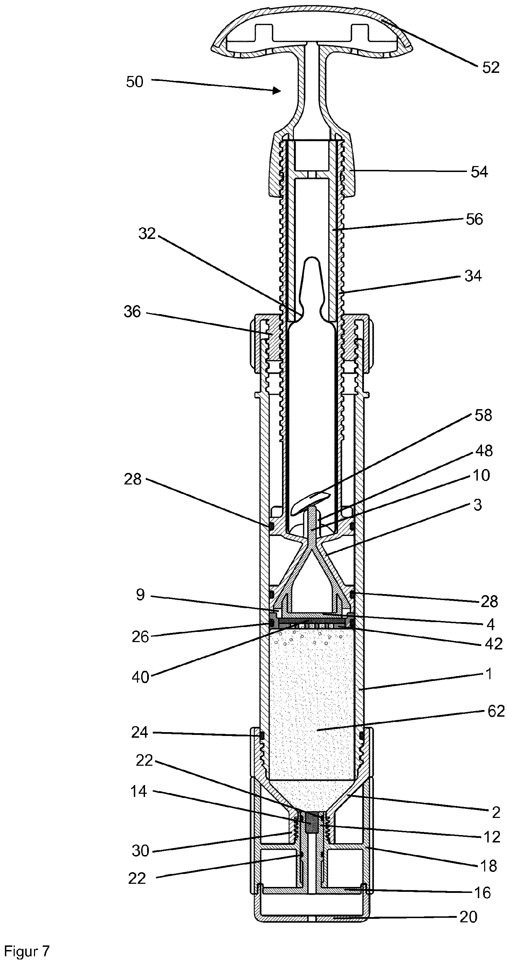

Gemäß einer bevorzugten Ausführung kann an einer der Vorderseite der Kartusche 1 gegenüberliegenden Rückseite der Kartusche 1 im Innenraum der Kartusche 1 ein Förderkolben 3 angeordnet sein, der axial im Innenraum der Kartusche 1 in Richtung der Vorderseite der Kartusche 1 beweglich gelagert ist. Zwischen dem Förderkolben 3 und der Vorderseite der Kartusche 1 kann ein Austragskolben 4 angeordnet sein. Der Förderkolben 3 und der Austragskolben 4 können zumindest teilweise aus Kunststoff gefertigt sein. Zwischen dem Austragskolben 4 und der Vorderseite der Kartusche 1 beziehungsweise dem Kartuschenkopf 2 ist ein erster Hohlraum 60 im Inneren der Vorrichtung gebildet (siehe Figur 2). In dem ersten Hohlraum 60 kann ein Zementpulver 5 als eine der Ausgangskomponenten des zu fertigenden Knochenzementteigs 62 (siehe Figuren 7 bis 9) enthalten sein. Bevorzugt ist das Zementpulver 5 zwischen dem Austragskolben 4 und dem Kartuschenkopf 2 im Kartuschenkopf 2 und in dem Innenraum der Kartusche 1 im ersten Hohlraum 60 eingepresst oder zumindest kompakt gelagert, um ein Einleiten und Verteilen einer Monomerflüssigkeit 6 in dem Zementpulver 5 durch Nutzung von Kapillarkräften zwischen den Zementpulverpartikeln zu vereinfachen.

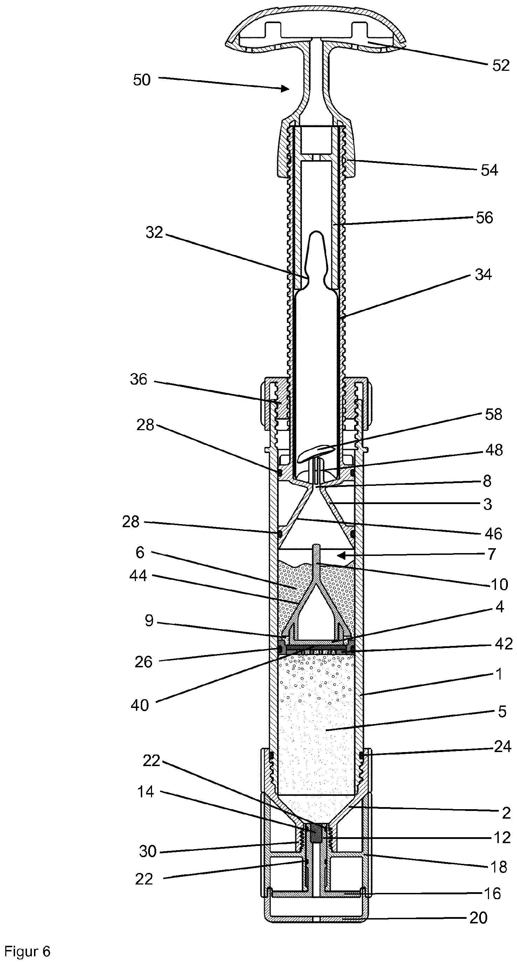

Von dem Austragskolben 4 und dem Förderkolben 3 und den Innenwänden der Kartusche 1 kann im Innenraum der Kartusche 1 ein zweiter Hohlraum 7 begrenzt sein. Eine Fluidöffnung 8 kann an der Spitze des trichterförmigen Förderkolbens 3 angeordnet sein, durch die die Monomerflüssigkeit 6 in den zweiten Hohlraum 7 eingeleitet werden kann. Die Fluidöffnung 8 kann als durch den Förderkolben 3 durchgehende zylindrische Bohrung realisiert werden. In dem Austragskolben 4 kann eine am Außenumfang des Austragskolbens 4 beginnende Durchführung 9 vorgesehen sein, durch die die Monomerflüssigkeit 6 aus dem zweiten Hohlraum 7 zu dem Zementpulver 5 in dem ersten Hohlraum 60 fließen oder geleitet werden kann (siehe Figur 6).

Der Austragskolben 4 kann an seiner Rückseite eine kegelförmige Oberfläche 44 aufweisen, die eine Negativform zu einer trichterförmigen Oberfläche 46 in dem trichterförmigen Förderkolben 3 bildet. Dabei kann bevorzugt vorgesehen sein, dass der Austragskolben 4 mit seiner kegelförmigen Oberfläche 44 flächenbündig an der trichterförmigen Oberfläche 46 des Förderkolbens 3 anliegen kann, wenn der Förderkolben 3 gegen den Austragskolben 4 gedrückt ist (siehe Figuren 7 bis 9). Das Volumen des zweiten Hohlraums 7 kann dabei auf null reduziert sein, so dass der zweiten Hohlraum 7 in diesem Zustand verschwunden ist.

Gemäß einer bevorzugten Ausführung kann vorgesehen sein, dass an der Spitze der kegelförmigen Oberfläche 44 an der Rückseite des Austragskolbens 4 ein Verschlussmittel 10 in Form eines zylindrischen Stifts angeordnet ist. Der Außenumfang des Verschlussmittels 10 kann dabei zu der Innenwand der Fluidöffnung 8 passen, so dass die Fluidöffnung 8 mit dem Verschlussmittel 10 gasdicht und fluiddicht verschließbar ist. Dazu kann der Außenumfang des Verschlussmittels 10 genauso groß oder etwas größer als der Innenumfang der Fluidöffnung 8 sein. Das Verschlussmittel 10 kann dabei derart langestreckt sein, dass es die Fluidöffnung 8 ab Unterschreiten eines Mindestabstands zwischen dem Austragskolben 4 und dem Förderkolben 3 verschließt. Dabei kann das Verschlussmittel 10 vorzugsweise durch die Fluidöffnung 8 hindurch geschoben werden, wenn der Förderkolben 3 in Richtung des Austragskolbens 4 gedrückt wird. Besonders bevorzugt kann das Verschlussmittel 10 in jeder Stellung des Förderkolbens 3 relativ zum Austragskolben 4 ab Unterschreiten eines Mindestabstands zwischen dem Austragskolben 4 und dem Förderkolben 3 die Fluidöffnung 8 verschließen.

Gemäß einer bevorzugten Ausführung können die Fluidöffnung 8 und das Verschlussmittel 10 entlang oder parallel zur Zylinderachse des zylindrischen Innenraums der Kartusche 1 angeordnet sein und miteinander fluchten.

Für den verständigen Fachmann ist dabei klar, dass diese Ausführung mit einer zentralen Fluidöffnung 8 im Förderkolben 4 ohne weiteres auf eine nicht zentrale Fluidöffnung übertragbar ist. Ebenso kann ein nicht auf der Zylinderachse des Innenraums der Kartusche 1 angeordnetes Verschlussmittel realisiert werden. Ferner können ohne weiteres mehrere Fluidöffnungen zum Durchleiten der Monomerflüssigkeit 6 im Förderkolben 3 vorgesehen sein. Dabei können auch ohne weiteres mehrere Verschlussmittel an der Rückseite des Austragskolbens 4 angeordnet sein. Ein für die Realisierung der vorliegenden Erfindung wichtiger und zentraler Aspekt ist darin zu sehen, dass alle gegebenenfalls vorhandenen Fluidöffnungen von den gegebenenfalls vorhandenen Verschlussmitteln verschließbar sind, bevor der Förderkolben 3 und der Austragskolben 4 aneinander anliegen und das Volumen des zweiten Hohlraums 7 auf null reduziert ist, wobei alle gegebenenfalls vorhandenen Fluidöffnungen von den gegebenenfalls vorhandenen Verschlussmitteln verschlossen bleiben, wenn der Förderkolben 3 weiter in Richtung des Austragskolbens 4 getrieben wird. Diesen Aspekt beherzigend, kann der Fachmann ohne weiteres andere ähnliche oder gleichwirkende Verschlussmöglichkeiten für die Fluidöffnungen finden, die als im Rahmen der vorliegenden Erfindung angesehen werden.

Die Austragsöffnung im Kartuschenkopf 2 kann zunächst mit einem Stopfen 12 verschlossen sein (siehe Figuren 1 bis 8). In dem Stopfen 12 kann ein Durchgang angeordnet sein, durch den Gase aus dem ersten Hohlraum 60 evakuiert und eingeleitet werden können. Um ein Austreten von Zementpulver 5 zu vermeiden, kann vorgesehen sein, dass in dem Durchgang ein Porenfilter 14 angeordnet ist, der für das Zementpulver 5 undurchlässig ist aber für Gase durchlässig ist. An dem Stopfen 12 kann ein farbiges Markierungsmittel 16 in Form einer farbigen Ringscheibe vorgesehen sein, an dem eine Bewegung des Stopfens 12 gegen die Austragsöffnung optisch erkennbar ist. Das farbige Markierungsmittel 16 kann hierzu beispielsweise rot sein oder eine andere Signalfarbe haben. Es sind aber auch andere (auch nicht optische) Methoden vorstellbar, eine Bewegung des Stopfens 12 gegen die Austragsöffnung für den Anwender kenntlich zu machen.

An dem Kartuschenkopf 2 kann ein Standfuß 18 mit einem unteren Standfußteil 20 angeordnet sein. Der Stopfen 12 kann vorzugsweise gegen den Standfuß 18 beweglich sein. Der untere Standfußteil 20 kann bevorzugt transparent sein, damit die Position des Markierungsmittels 16 erkennbar ist. Besonders bevorzugt kann das Markierungsmittel 18 zunächst in einem nicht transparenten Teil des Standfußes 18 verborgen sein und wird erst bei einer Bewegung des Stopfens 12 aus der Austragsöffnung hinaus in den transparenten unteren Standfußteil 20 bewegt, wo es dann durch den transparenten unteren Standfußteil 20 sichtbar ist.

Um ein Austreten von Monomerflüssigkeit 6 und Knochenzementteig 62 zu verhindern und/oder um den ersten Hohlraum 60 von dem zweiten Hohlraum 7 oder andere Bereiche voneinander abzudichten, können Abdichtungen vorgesehen sein. So können zwei Dichtungsringe 22 in zwei umlaufenden Nuten an einem Außenumfang des Stopfens 12 angeordnet sein, die den Stopfen 12 gegen die Austragsöffnung und/oder gegen den Standfuß 18 abdichten. Es kann ein Dichtungsring 24 vorgesehen sein, der in einer umlaufenden Nut an der Außenseite der Kartusche 1 angeordnet ist und den Kartuschenkopf 2 gegen die Kartusche 1 abdichtet. Es kann ein Dichtungsring 26 in einer umlaufenden Nut am Außenumfang des Austragskolbens 4 angeordnet sein, der die Austragskolben 4 gegen die Innenwand der Kartusche 1 abdichtet. Es können zwei Dichtungsringe 28 in zwei umlaufenden Nuten am Außenumfang des Förderkolbens 3 und am Außenumfang eines Behälters 34, der an der Rückseite des Förderkolbens 3 angeordnet sein kann, angeordnet sein, die den Förderkolben 3 und den Behälter 34 gegen die Innenwand der Kartusche 1 abdichten.

An der Spitze des Kartuschenkopfs 2 kann ein Stutzen 30 ausgebildet sein, der die Austragsöffnung umschließt. In dem Stutzen 30 kann ein Innengewinde vorgesehen sein, in das ein Austragsrohr (nicht gezeigt) mit einem passenden Außengewinde eingeschraubt werden kann. In das gleiche Innengewinde oder an einem anderen Befestigungsmittel des Stutzens 30 kann der Standfuß 18 mit einem passenden Außengewinde oder einem anderen passenden Gegenbefestigungsmittel an dem Stutzen 30 lösbar befestigt sein.

Um ein vollständiges Full-Prepacked-Mischsystem zu erhalten, kann ein Monomerflüssigkeitsbehälter 32, beispielsweise in Form einer aufbrechbaren Ampulle aus Glas oder Kunststoff, in dem Behälter 34 angeordnet sein, der hierzu an der Rückseite des Förderkolbens 3 angeordnet sein kann. In dem Monomerflüssigkeitsbehälter 32 kann die Monomerflüssigkeit 6 enthalten sein. Der Innenraum des Behälters 34 kann über die Fluidöffnung 8 mit dem zweiten Hohlraum 7 flüssigkeitsdurchlässig verbunden sein. Dadurch kann eine in dem Behälter 34 freigesetzte Monomerflüssigkeit 6 aus dem Behälter 34 in den zweiten Hohlraum 7 fließen, wenn die Vorrichtung mit dem Kartuschenkopf 2 nach unten gehalten oder aufgestellt ist.

An einem dem Kartuschenkopf 2 gegenüberliegenden rückseitigen Ende der Kartusche 1 kann eine Ringhülse 36 mit einem Innengewinde befestigt sein. Diese erleichtert die Montage der Vorrichtung, wenn sie als zur Kartusche 1 separates Teil vorliegt. Die Ringhülse 36 kann hierzu mit einem passenden Außengewinde in ein Innengewinde an dem rückseitigen Ende der Kartusche 1 geschraubt sein.

Es kann gemäß einer bevorzugten Weiterbildung der vorliegenden Erfindung vorgesehen sein, dass der Behälter 34 an seinem Außenumfang ein Außengewinde aufweist. Dieses Außengewinde passt besonders bevorzugt zu dem Innengewinde der Ringhülse 36 oder zu einem Innengewinde an dem rückseitigen Ende der Kartusche 1. Dadurch kann der Behälter 34 in die Kartusche 1 eingeschraubt werden. Dies kann einen Vortrieb des Förderkolbens 3 im Innenraum der Kartusche 1 ermöglichen. Theoretisch kann als Alternative zum Behälter 34 auch ein Zylinder (nicht gezeigt) mit einem passenden Außengewinde an der Rückseite des Förderkolbens 3 befestigt oder auch lose angeordnet sein, so dass durch Einschrauben des Zylinders der Förderkolben 3 und über den Förderkolben 3 auch der Austragskolben 4 im Innenraum der Kartusche 1 in Richtung des Kartuschenkopfs 2 zu drücken ist.

Um eine vorzeitige oder ungewollte Bewegung des Behälters 34 und/oder der Ringhülse 36 gegen die Kartusche 1 zu verhindern, kann ein abziehbares Sicherungsmittel 38 vorgesehen sein. Das Sicherungsmittel 38 kann hierzu als Spange geformt sein, die am Außenumfang des Behälters 34 oder einer anderen rückseitigen Verlängerung des Förderkolbens 3 eingreift und sich am rückseitigen Ende der Kartusche 1 oder an der Ringhülse 36 abstützt.

Es kann bevorzugt vorgesehen sein, dass in oder an dem Austragskolben 4 ein Porenfilter 40 angeordnet ist. Mit dem Porenfilter 40 können die Durchführungen 9 abgedeckt sein. Hierdurch wird verhindert, dass Zementpulver 5 aus dem ersten Hohlraum 60 in die Durchführungen 9 oder in den zweiten Hohlraum 7 eindringt, dort bei Reaktion mit der Monomerflüssigkeit 6 eine gelartige Barriere bildet und so einer Verteilung der Monomerflüssigkeit 6 in dem Zementpulver 5 entgegenwirkt.

Der Porenfilter 40 kann bevorzugt eine Kreisscheibe sein. Der Porenfilter 40 kann mit einer Scheibe 42 abgedeckt und in dem Austragskolben 4 befestigt sein. Um eine Durchleitung und Verteilung der Monomerflüssigkeit 6 in den ersten Hohlraum 60 und in dem Zementpulver 5 zu ermöglichen beziehungsweise zu erleichtern, können in der Scheibe 42 eine Vielzahl von Bohrungen oder Löchern vorgesehen sein (siehe Figur 2).

Im Innenraum des Behälters 34 können mehrere Stifte 48 angeordnet sein. Diese Stifte 48 können an einem Kartuschenboden 58 des Monomerflüssigkeitsbehälters 32 anliegen. Die Stifte 48 können dazu verwendet werden, den Monomerflüssigkeitsbehälter 32 am Kartuschenboden 58 aufzubrechen oder eine Monomerflüssigkeitsbehälter 32 aufzustechen oder aufzuschneiden. Bevorzugt können die Stifte 48 um die Einmündung der Fluidöffnung 8 herum angeordnet sein. Die Stifte 48 können so voneinander beabstandet werden, dass sie eine Verlängerung der Fluidöffnung 8 nicht abdecken, um ein Durchtreten des Verschlussmittels 10 zu ermöglichen.

An der vom Förderkolben 3 abgewandten Rückseite des Behälters 34 kann ein Öffnungsmittel 50 zum Öffnen des Monomerflüssigkeitsbehälters 32 angeordnet sein. Das Öffnungsmittel 50 kann hierzu einen manuell bedienbaren Griff 52 und eine Kappe 54 aufweisen. Es kann vorgesehen sein, dass die Kappe 54 gegen den Behälter 34 beweglich gelagert ist, wobei vorzugsweise die Kappe 54 auf das Außengewinde des Behälters 34 aufschraubbar ist. Der Behälter 34 kann an seiner Rückseite offen sein. Im Inneren des Behälters 34 kann eine Hülse 56 in Form eines Rohrstücks als Teil des Öffnungsmittels 50 beweglich gegen den Behälter 34 gelagert sein. Durch eine Bewegung der Hülse 56 in den Behälter 34 hinein kann der Monomerflüssigkeitsbehälter 32 in dem Behälter 34 geöffnet werden. Bevorzugt wird eine Ampulle als Monomerflüssigkeitsbehälter 32 mit dem Kartuschenboden 58 derart auf die Stifte 48 gedrückt, dass der Kartuschenboden 58 aufbricht oder abbricht und dadurch die Ampulle geöffnet ist (siehe Figur 4). Auf diese Weise kann die Monomerflüssigkeit 6 aus dem Monomerflüssigkeitsbehälter 32 im Inneren des Behälters 34 freigesetzt werden.

Im Folgenden wird der Ablauf eines erfindungsgemäßen Verfahrens am Beispiel der erfindungsgemäßen Vorrichtung anhand der Figuren 1 bis 10 erläutert.

Die Vorrichtung befindet sich zunächst im Ursprungszustand oder Lagerungszustand, wie er in den Figuren 1 bis 3 gezeigt ist. In diesem Zustand kann die Vorrichtung durch geeignete Öffnungen evakuiert und mit Ethylenoxid sterilisiert werden.

Als nächstes kann die Vorrichtung mit dem Standfuß 18 auf einer ebenen Unterlage aufgestellt werden. Danach kann das Öffnungsmittel 50 auf den Behälter 34 geschraubt werden, bis der Kartuschenboden 58 des Monomerflüssigkeitsbehälters 32 aufbricht. Durch das Aufbrechen des Monomerflüssigkeitsbehälters 32 wird die darin enthaltene Monomerflüssigkeit 6 freigesetzt und kann aus dem Behälter 34 durch die Fluidöffnung 8 in den zweiten Hohlraum 7 fließen. Diese Situation ist in Figur 4 dargestellt.

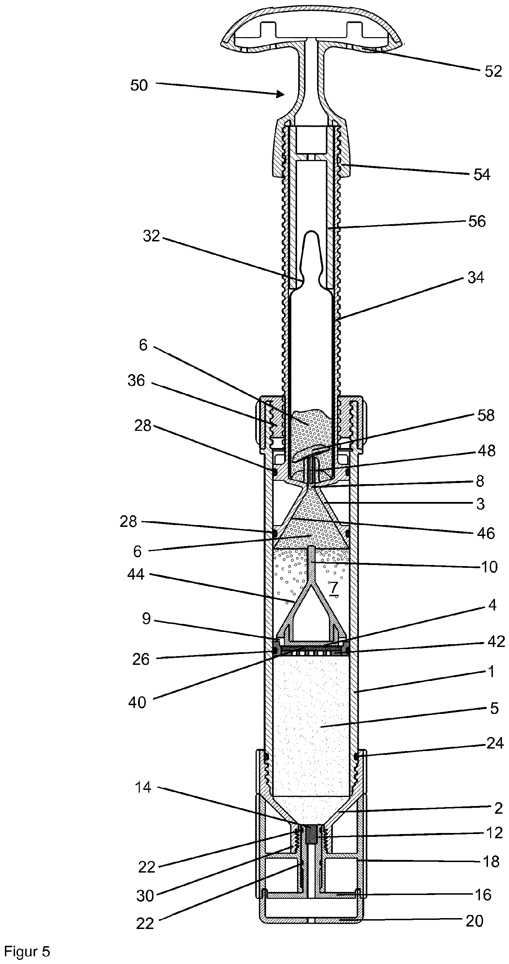

Anschließend kann das Sicherungsmittel 38 abgezogen werden. Der Behälter 34 kann nun tiefer in die Kartusche 1 eingeschoben werden, bis das Außengewinde am Behälter 34 auf das Innengewinde an der Ringhülse 36 trifft und eine weitere Bewegung des Behälters 34 in die Kartusche 1 hinein blockiert. Diese Situation ist in Figur 5 dargestellt. Mit dem Behälter 34 kann auch der Förderkolben 3 in dem Innenraum der Kartusche 1 in Richtung des Austragskolbens 4 gedrückt werden. Es kann dabei vorgesehen sein, dass das Verschlussmittel in dem in Figur 5 gezeigten Zustand noch nicht die Fluidöffnung 8 verschließen kann, um ein weiteres Einströmen der Monomerflüssigkeit 6 durch die Fluidöffnung 8 in den zweiten Hohlraum 7 zu ermöglichen. Das Verschlussmittel 10 kann zu diesem Zweck einfach ausgehend von der Rückseite des Austragskolbens 4 nicht lang genug aufgebaut sein. Gleichwohl kann das Volumen des zweiten Hohlraums 7 durch die Bewegung des Förderkolbens 3 in Richtung des Austragskolbens 4 verringert werden.

Die Ringhülse 36 kann nun nach oben von dem Kartuschenkopf 10 der Kartusche 1 weg geschraubt werden, bis das Innengewinde der Ringhülse 36 in das Außengewinde des Behälters 34 greift. Währenddessen ist die Monomerflüssigkeit 6 vollständig aus dem Behälter 34 in den zweiten Hohlraum 7 geflossen und kann bereits beginnen, durch die Durchführung 9 im Austragskolben 4 und gegebenenfalls durch die Porenscheibe 40 und die Scheibe 42 hindurch in den ersten Hohlraum 60 fließen. Diese Situation ist in Figur 6 dargestellt.

Sobald die Monomerflüssigkeit 6 in den ersten Hohlraum 60 fließt, verteilt sie sich aufgrund von Kapillarkräften zwischen den Pulverteilchen des Zementpulvers 5 und dadurch in dem Zementpulver 5. Als nächstes kann der Behälter 34 tiefer in die Kartusche 1 eingeschraubt werden, in dem der Behälter 34 gegen die Ringhülse 36 beziehungsweise gegen die Kartusche 1 gedreht wird. Dabei kann der Förderkolben 3 immer weiter in Richtung des Austragskolbens 4 gedrückt werden. Ab einem Mindestabstand zwischen dem Förderkolben 3 und dem Austragskolben 4 kann bei einer weiteren Bewegung des Förderkolbens 3 zum Austragskolben 4 hin das Verschlussmittel 10 in die Fluidöffnung 8 eindringen und die Fluidöffnung 8 flüssigkeitsdicht und gasdicht verschließen. Es kann vorgesehen sein, dass zu diesem Zeitpunkt keine Gaseinschlüsse beziehungsweise keine Lufteinschlüsse mehr im zweiten Hohlraum 7 enthalten sind, weil diese zuvor alle aus der Fluidöffnung 8 nach oben entweichen konnten (siehe Figur 10). Hierfür kann die trichterförmige Oberfläche 46 bei ausreichend starker Steigung sorgen. Die Fluidöffnung 8 kann gemäß einer bevorzugten Ausführung bei einer weiteren Bewegung des Förderkolbens 3 zum Austragskolben 4 hin verschlossen bleiben.

Der Behälter 34 kann nun noch weiter in die Kartusche 1 eingeschraubt werden. Dabei wird die Monomerflüssigkeit 6 aus dem zweiten Hohlraum 7 in das Zementpulver 5 im ersten Hohlraum 60 gepresst und verteilt sich dort. Schließlich kann der Förderkolben 3 auf den Austragskolben 4 auftreffen, so dass der Förderkolben 3 mit seiner trichterförmigen Oberfläche 46 an der passenden kegelförmigen Oberfläche 44 an der Rückseite des Austragskolbens 4 anliegt, insbesondere flächenbündig anliegt. Währenddessen quilt das Zementpulver 5 mit der Monomerflüssigkeit 6 an und bildet den Knochenzementteig 62. Diese Situation ist in Figur 7 dargestellt.

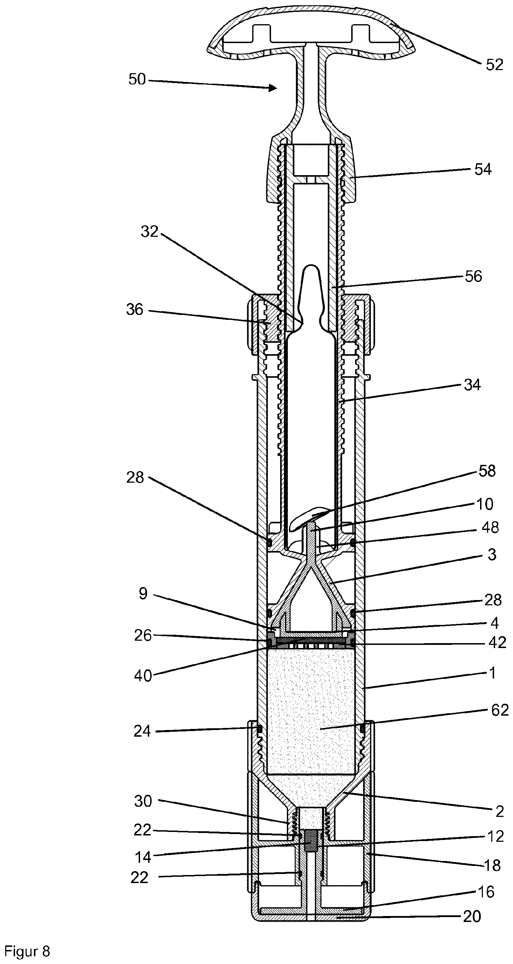

Der Behälter 32 kann noch tiefer in die Kartusche 1 eingeschraubt werden, wenn der Knochenzement 62 bis zur Austragsöffnung gebildet ist beziehungsweise das Zementpulver 5 bis zur Austragsöffnung mit der Monomerflüssigkeit 6 benetzt ist, da erst dann ein fließfähiges Material im ersten Hohlraum 60 erhalten wird, das in die Austragsöffnung gedrückt werden kann. Durch die Bewegung des Knochenzementteigs 62 kann der Stopfen 12 in der Austragsöffnung bewegt werden. Dies kann durch eine Verschiebung des Markierungsmittels 16 in den transparenten Standfußteil 20 optisch erkannt werden, so dass der Anwender weiß, dass der Knochenzementteig 62 nun einsatzbereit ist. Diese Situation ist in Figur 8 dargestellt.

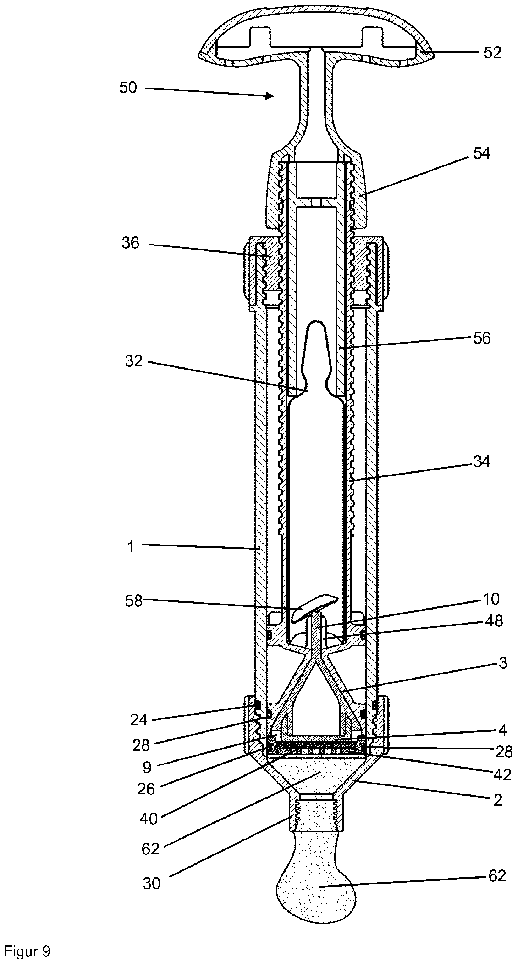

Als nächstes kann der Standfuß 18 mit dem Stopfen 12 aus der Austragsöffnung gelöst werden. Daran anschließend kann der Knochenzementteig 62 durch weiteres Einschrauben des Behälters 34 und damit einhergehend durch weiteres Vortreiben des Förderkolbens 3 und des Austragskolbens 4 in Richtung des Austragsöffnung aus der Austragsöffnung ausgetragen werden. Diese Situation ist in Figur 9 dargestellt. Es kann vorgesehen sein, dass der Anwender ein Austragsrohr (nicht gezeigt) zum Applizieren des Knochenzementteigs 62 an dem Stutzen 30 oder an einer anderen Stelle anschließt.

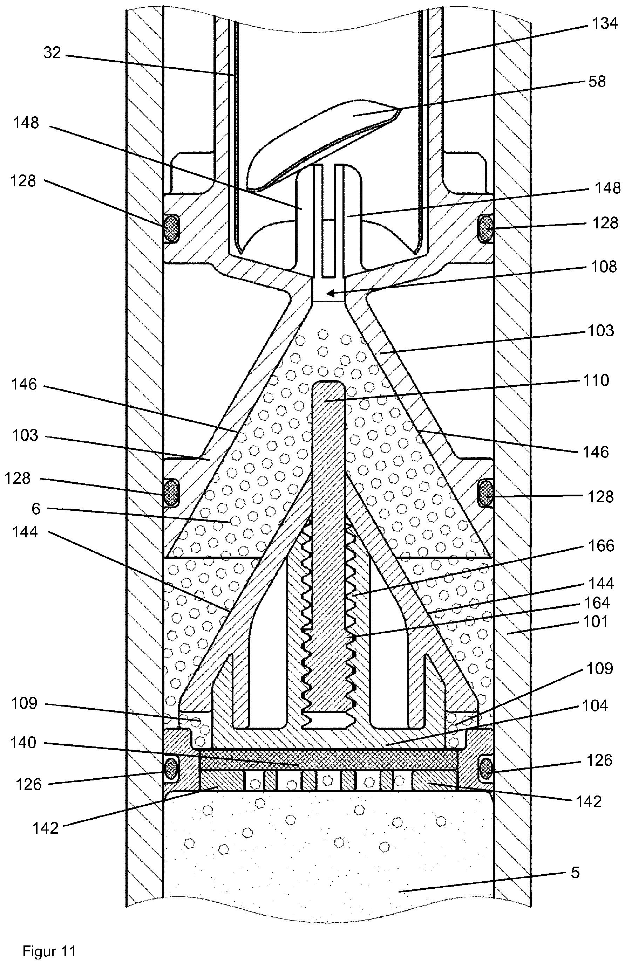

Figur 11 zeigt eine schematische Querschnittansicht einer Ausschnittvergrößerung einer alternativen erfindungsgemäßen Vorrichtung mit einem variabel einstellbaren Verschlussmittel 110. Der in Figur 11 gezeigte Ausschnitt entspricht dem in Figur 10 gezeigten Ausschnitt, um den Unterschied der beiden Ausführungsformen deutlich zu machen. Das zweite Ausführungsbeispiel nach Figur 11 entspricht dem ersten Ausführungsbeispiel nach den Figuren 1 bis 10, bis auf die variable Einstellmöglichkeit des Verschlussmittels 110.

Gemäß einer bevorzugten Ausführung der vorliegenden Erfindung nach dem zweiten Ausführungsbeispiel hat die Vorrichtung eine röhrenförmigen Kartusche 101 aus einem Kunststoff mit einem zylindrischen Innenraum. Die Kartusche 101 kann an ihrer Vorderseite mit einem trichterförmigen Kartuschenkopf (nicht zu sehen) aus Kunststoff verschlossen sein. Die Vorderseite der Kartusche 101 ist in Figur 11 unten. An der Spitze des trichterförmigen Kartuschenkopfs kann eine zentrale Austragsöffnung angeordnet sein, die im Ausgangszustand aber zunächst verschlossen sein kann. Der Kartuschenkopf kann gemäß einer alternativen Ausführung aber auch eine flache Kappe sein oder eine andere Form aufweisen.

Gemäß einer bevorzugten Ausführung kann an einer der Vorderseite der Kartusche 101 gegenüberliegenden Rückseite im Innenraum der Kartusche 101 ein Förderkolben 103 angeordnet sein, der axial im Innenraum der Kartusche 101 in Richtung der Vorderseite der Kartusche 101 beweglich gelagert ist. Zwischen dem Förderkolben 103 und der Vorderseite der Kartusche 101 kann ein Austragskolben 104 angeordnet sein. Der Förderkolben 103 und der Austragskolben 104 können zumindest teilweise aus Kunststoff gefertigt sein. Zwischen dem Austragskolben 104 und der Vorderseite der Kartusche 101 beziehungsweise dem Kartuschenkopf ist ein erster Hohlraum im Inneren der Vorrichtung gebildet. In dem ersten Hohlraum kann ein Zementpulver 5 als eine der Ausgangskomponenten des zu fertigenden Knochenzementteigs enthalten sein. Bevorzugt ist das Zementpulver 5 zwischen dem Austragskolben 104 und dem Kartuschenkopf im Kartuschenkopf und in dem Innenraum der Kartusche 101 im ersten Hohlraum 60 eingepresst oder zumindest kompakt gelagert, um ein Einleiten und Verteilen einer Monomerflüssigkeit 6 in dem Zementpulver 5 durch Nutzung von Kapillarkräften zwischen den Zementpulverpartikeln zu vereinfachen.

Von dem Austragskolben 104 und dem Förderkolben 103 und den Innenwänden der Kartusche 101 kann im Innenraum der Kartusche 101 ein zweiter Hohlraum 107 begrenzt sein. Eine Fluidöffnung 108 kann an der Spitze des trichterförmigen Förderkolbens 103 angeordnet sein, durch die die Monomerflüssigkeit 106 in den zweiten Hohlraum 107 eingeleitet werden kann. Die Fluidöffnung 108 kann als durch den Förderkolben 103 durchgehende zylindrische Bohrung realisiert werden. In dem Austragskolben 104 kann eine am Außenumfang des Austragskolbens 104 beginnende Durchführung 109 vorgesehen sein, durch die die Monomerflüssigkeit 6 aus dem zweiten Hohlraum 107 zu dem Zementpulver 5 in dem ersten Hohlraum fließen oder geleitet werden kann.

Der Austragskolben 104 kann an seiner Rückseite eine kegelförmige Oberfläche 144 aufweisen, die eine Negativform zu einer trichterförmigen Oberfläche 146 in dem trichterförmigen Förderkolben 103 bildet. Dabei kann bevorzugt vorgesehen sein, dass der Austragskolben 104 mit seiner kegelförmigen Oberfläche 144 flächenbündig an der trichterförmigen Oberfläche 146 des Förderkolbens 103 anliegen kann, wenn der Förderkolben 103 gegen den Austragskolben 104 gedrückt ist. Das Volumen des zweiten Hohlraums 107 kann dabei auf null reduziert sein, so dass der zweiten Hohlraum 107 in diesem Zustand verschwunden ist.

Gemäß einer bevorzugten Ausführung kann vorgesehen sein, dass an der Spitze der kegelförmigen Oberfläche 144 an der Rückseite des Austragskolbens 104 das Verschlussmittel 110 in Form eines zylindrischen Stifts angeordnet ist. Der Außenumfang des Verschlussmittels 110 kann dabei zu der Innenwand der Fluidöffnung 108 passen, so dass die Fluidöffnung 108 mit dem Verschlussmittel 110 gasdicht und fluiddicht verschließbar ist. Dazu kann der Außenumfang des Verschlussmittels 110 genauso groß oder etwas größer als der Innenumfang der Fluidöffnung 108 sein. Das Verschlussmittel 110 kann dabei derart langestreckt sein, dass es die Fluidöffnung 108 ab Unterschreiten eines Mindestabstands zwischen dem Austragskolben 104 und dem Förderkolben 103 verschließt. Dabei kann das Verschlussmittel 110 vorzugsweise durch die Fluidöffnung 108 hindurch geschoben werden, wenn der Förderkolben 103 in Richtung des Austragskolbens 104 gedrückt wird. Besonders bevorzugt kann das Verschlussmittel 110 in jeder Stellung des Förderkolbens 103 relativ zum Austragskolben 104 ab Unterschreiten eines Mindestabstands zwischen dem Austragskolben 104 und dem Förderkolben 103 die Fluidöffnung 108 verschließen.

Gemäß einer bevorzugten Ausführung können die Fluidöffnung 108 und das Verschlussmittel 110 entlang oder parallel zur Zylinderachse des zylindrischen Innenraums der Kartusche 101 angeordnet sein und miteinander fluchten.

Für den verständigen Fachmann ist dabei klar, dass auch diese Ausführung mit einer zentralen Fluidöffnung 108 im Förderkolben 104 ohne weiteres auf eine nicht zentrale Fluidöffnung übertragbar ist. Ebenso kann ein nicht auf der Zylinderachse des Innenraums der Kartusche 101 angeordnetes Verschlussmittel realisiert werden. Ferner können ohne weiteres mehrere Fluidöffnungen zum Durchleiten der Monomerflüssigkeit 6 im Förderkolben 103 vorgesehen sein. Dabei können auch ohne weiteres mehrere Verschlussmittel an der Rückseite des Austragskolbens 104 angeordnet sein. Ein für die Realisierung der vorliegenden Erfindung wichtiger und zentraler Aspekt ist darin zu sehen, dass alle gegebenenfalls vorhandenen Fluidöffnungen von den gegebenenfalls vorhandenen Verschlussmitteln verschließbar sind, bevor der Förderkolben 103 und der Austragskolben 104 aneinander anliegen und das Volumen des zweiten Hohlraums 107 auf null reduziert ist, wobei alle gegebenenfalls vorhandenen Fluidöffnungen von den gegebenenfalls vorhandenen Verschlussmitteln verschlossen bleiben, wenn der Förderkolben 103 weiter in Richtung des Austragskolbens 104 getrieben wird. Diesen Aspekt beherzigend, kann der Fachmann ohne weiteres andere ähnliche oder gleichwirkende Verschlussmöglichkeiten für die Fluidöffnungen finden, die als im Rahmen der vorliegenden Erfindung angesehen werden.

Die Austragsöffnung im Kartuschenkopf kann zunächst mit einem Stopfen (nicht gezeigt) verschlossen sein. Um ein Austreten von Monomerflüssigkeit 6 und Knochenzementteig zu verhindern und/oder um den ersten Hohlraum von dem zweiten Hohlraum 107 oder andere Bereiche voneinander abzudichten, können Abdichtungen vorgesehen sein. Hierzu kann ein Dichtungsring 126 in einer umlaufenden Nut am Außenumfang des Austragskolbens 104 angeordnet sein, der die Austragskolben 104 gegen die Innenwand der Kartusche 101 abdichtet. Es können zwei Dichtungsringe 128 in zwei umlaufenden Nuten am Außenumfang des Förderkolbens 103 und am Außenumfang eines Behälters 134, der an der Rückseite des Förderkolbens 103 angeordnet sein kann, angeordnet sein, die den Förderkolben 103 und den Behälter 134 gegen die Innenwand der Kartusche 101 abdichten.

Um ein vollständiges Full-Prepacked-Mischsystem zu erhalten, kann ein Monomerflüssigkeitsbehälter 32, beispielsweise in Form einer aufbrechbaren Ampulle aus Glas oder Kunststoff, in dem Behälter 134 angeordnet sein, der hierzu an der Rückseite des Förderkolbens 103 angeordnet sein kann. In dem Monomerflüssigkeitsbehälter 32 kann die Monomerflüssigkeit 6 enthalten sein. Der Innenraum des Behälters 134 kann über die Fluidöffnung 108 mit dem zweiten Hohlraum 107 flüssigkeitsdurchlässig verbunden sein. Dadurch kann eine in dem Behälter 134 freigesetzte Monomerflüssigkeit 6 aus dem Behälter 134 in den zweiten Hohlraum 107 fließen, wenn die Vorrichtung mit dem Kartuschenkopf nach unten gehalten oder aufgestellt ist.

An einem dem Kartuschenkopf gegenüberliegenden rückseitigen Ende der Kartusche 101 kann eine Ringhülse 136 mit einem Innengewinde befestigt sein. Diese erleichtert die Montage der Vorrichtung, wenn sie als zur Kartusche 101 separates Teil vorliegt. Die Ringhülse 136 kann hierzu mit einem passenden Außengewinde in ein Innengewinde an dem rückseitigen Ende der Kartusche 101 geschraubt sein.

Es kann gemäß einer bevorzugten Weiterbildung der vorliegenden Erfindung vorgesehen sein, dass der Behälter 134 an seinem Außenumfang ein Außengewinde aufweist. Dieses Außengewinde passt besonders bevorzugt zu dem Innengewinde der Ringhülse 136 oder zu einem Innengewinde an dem rückseitigen Ende der Kartusche 101. Dadurch kann der Behälter 134 in die Kartusche 101 eingeschraubt werden. Dies kann einen Vortrieb des Förderkolbens 103 im Innenraum der Kartusche 101 ermöglichen. Theoretisch kann als Alternative zum Behälter 134 auch ein Zylinder (nicht gezeigt) mit einem passenden Außengewinde an der Rückseite des Förderkolbens 103 befestigt oder auch lose angeordnet sein, so dass durch Einschrauben des Zylinders der Förderkolben 103 und über den Förderkolben 103 auch der Austragskolben 104 im Innenraum der Kartusche 101 in Richtung des Kartuschenkopfs zu drücken ist.

Es kann bevorzugt vorgesehen sein, dass in oder an dem Austragskolben 104 ein Porenfilter 140 angeordnet ist. Mit dem Porenfilter 140 können die Durchführungen 109 abgedeckt sein. Hierdurch wird verhindert, dass Zementpulver 5 aus dem ersten Hohlraum in die Durchführungen 109 oder in den zweiten Hohlraum 107 eindringt, dort bei Reaktion mit der Monomerflüssigkeit 6 eine gelartige Barriere bildet und so einer Verteilung der Monomerflüssigkeit 6 in dem Zementpulver 5 entgegenwirkt.

Der Porenfilter 140 kann bevorzugt eine Kreisscheibe sein. Der Porenfilter 140 kann mit einer Scheibe 142 abgedeckt und in dem Austragskolben 104 befestigt sein. Um eine Durchleitung und Verteilung der Monomerflüssigkeit 6 in den ersten Hohlraum und in dem Zementpulver 5 zu ermöglichen beziehungsweise zu erleichtern, können in der Scheibe 142 eine Vielzahl von Bohrungen oder Löchern vorgesehen sein.

Im Innenraum des Behälters 134 können mehrere Stifte 148 angeordnet sein. Diese Stifte 148 können an einem Kartuschenboden 58 des Monomerflüssigkeitsbehälters 32 anliegen. Die Stifte 148 können dazu verwendet werden, den Monomerflüssigkeitsbehälter 32 am Kartuschenboden 58 aufzubrechen oder eine Monomerflüssigkeitsbehälter 32 aufzustechen oder aufzuschneiden. Bevorzugt können die Stifte 148 um die Einmündung der Fluidöffnung 108 herum angeordnet sein. Die Stifte 148 können so voneinander beabstandet werden, dass sie eine Verlängerung der Fluidöffnung 108 nicht abdecken, um ein Durchtreten des Verschlussmittels 110 zu ermöglichen.

Im Gegensatz zum ersten Ausführungsbeispiel nach den Figuren 1 bis 10 kann bei dem zweiten Ausführungsbeispiel nach Figur 11 das Verschlussmittel 110 nicht starr mit dem Austragskolben 104 verbunden sein, sondern das Verschlussmittel 110 kann über ein Außengewinde 164 in ein Innengewinde an der Rückseite des Austragskolbens 104 geschraubt sein. Hierdurch kann eine variable Höhe des Verschlussmittels 110 eingestellt werden, so dass der Mindestabstand zwischen dem Förderkolben 103 und dem Austragskolben 104, ab dem die Fluidöffnung 108 von dem Verschlussmittel 110 verschlossen wird, eingestellt werden kann.

### Bezugszeichenliste

- 1, 101: Kartusche
- 2: Kartuschenkopf
- 3, 103: Förderkolben
- 4, 104: Austragskolben
- 5: Zementpulver
- 6: Monomerflüssigkeit
- 7: Zweiter Hohlraum
- 8, 108: Fluidöffnung
- 9, 109: Durchführung
- 10, 110: Verschlussmittel
- 12: Stopfen
- 14: Porenfilter
- 16: Markierungsmittel
- 18: Standfuß
- 20: Unterer Standfußteil
- 22: Dichtungsring
- 24: Dichtungsring
- 26, 126: Dichtungsring
- 28, 128: Dichtungsring
- 30: Stutzen
- 32: Monomerflüssigkeitsbehälter
- 34, 134: Behälter
- 36: Ringhülse
- 38: Sicherungsmittel
- 40, 140: Porenfilter
- 42, 142: Scheibe
- 44, 144: Kegelförmige Oberfläche
- 46, 146: Trichterförmige Oberfläche
- 48, 148: Stift
- 50: Öffnungsmittel
- 52: Griff
- 54: Kappe
- 56: Hülse
- 58: Kartuschenboden
- 60: Erster Hohlraum
- 62: Knochenzementteig
- 164: Außengewinde
- 166: Innengewinde

## Patentansprüche

1. Vorrichtung zum Herstellen eines Knochenzementteigs (62) aus einer Monomerflüssigkeit (6) und einem Zementpulver (5) als Ausgangskomponenten des Knochenzementteigs (62) und zum Austragen des Knochenzementteigs (62), die Vorrichtung aufweisend
eine Kartusche (1, 101) mit einem zylindrischen Innenraum,
einen Kartuschenkopf (2) mit einer Austragsöffnung zum Austreiben des Knochenzementteigs (62), wobei der Kartuschenkopf (2) die Kartusche (1, 101) an einer Vorderseite der Kartusche (1, 101) bis auf die Austragsöffnung verschließt, einen Förderkolben (3, 103), der im Innenraum der Kartusche (1, 101) angeordnet ist und der im Innenraum der Kartusche (1, 101) in Richtung der Austragsöffnung drückbar gelagert ist,
einen Austragskolben (4, 104), der im Innenraum der Kartusche (1, 101) zwischen der Austragsöffnung und dem Förderkolben (3, 103) angeordnet ist und der im Innenraum der Kartusche (1, 101) in Richtung der Austragsöffnung drückbar gelagert ist,
einen ersten Hohlraum (60), der von dem Kartuschenkopf (2), von Innenwänden der Kartusche (1, 101) und von dem Austragskolben (4, 104) begrenzt ist, wobei das Zementpulver (5) in dem ersten Hohlraum (60) angeordnet ist,
einen zweiten Hohlraum (7), der ein Teil des zylindrischen Innenraums der Kartusche (1, 101) ist, wobei der zweite Hohlraum (7) von dem Austragskolben (4, 104) und dem Förderkolben (3, 103) begrenzt ist,
eine Fluidöffnung (8, 108), die in dem Förderkolben (3, 103) angeordnet ist und durch die die Monomerflüssigkeit (6) in den zweiten Hohlraum (7) einleitbar ist,
eine Durchführung (9, 109), die in dem Austragskolben (4, 104) und/oder in der Wandung der Kartusche (1, 101) angeordnet ist und die den ersten Hohlraum (60) und den zweiten Hohlraum (7) für die Monomerflüssigkeit (6) durchlässig aber für das Zementpulver (5) undurchlässig miteinander verbindet, und
ein Verschlussmittel (10, 110), das an einer der Austragsöffnung abgewandten Rückseite des Austragskolbens (4, 104) angeordnet ist, wobei das Verschlussmittel (10, 110) von dem Austragskolben (4, 104) in Richtung des Förderkolbens (3, 103) absteht und von der Fluidöffnung (8, 108) in dem Förderkolben (3, 103) beabstandet ist, wobei mit dem Verschlussmittel (10, 110) die Fluidöffnung (8, 108) verschließbar ist, wenn der Förderkolben (3, 103) in Richtung der Austragsöffnung gedrückt wird, und wobei das Verschlussmittel (10, 110) derart in der mit dem Verschlussmittel (10, 110) verschlossenen Fluidöffnung (8, 108) beweglich ist, dass die Fluidöffnung (8, 108) verschlossen bleibt, während der Förderkolben (3, 103) weiter in Richtung des Austragskolbens (4, 104) drückbar ist.

2. Vorrichtung nach Anspruch 1, **dadurch gekennzeichnet, dass**
eine Vorderseite des Förderkolbens (3, 103), die dem Austragskolben (4, 104) zugewandt ist, und die Rückseite des Austragskolbens (4, 104) zueinander flächenbündig passende Formen aufweisen, so dass die Vorderseite des Förderkolbens (3, 103) an der Rückseite des Austragskolbens (4, 104) flächenbündig anliegt, wenn der Förderkolben (3, 103) gegen den Austragskolben (4, 104) gedrückt ist, wobei vorzugsweise das Volumen des zweiten Hohlraums (7) dadurch auf maximal 1% des Volumens des zweiten Hohlraums (7) in einem Ausgangszustand reduzierbar ist, wobei im Ausgangszustand das zumindest eine Verschlussmittel (10, 110) von der zumindest einen Fluidöffnung (8, 108) beabstandet ist.

3. Vorrichtung nach Anspruch 1 oder 2, **dadurch gekennzeichnet, dass**
eine Vorderseite des Förderkolbens (3, 103), die dem Austragskolben (4, 104) zugewandt ist, eine sich stetig in Richtung der Fluidöffnung (8, 108) verjüngende Oberfläche aufweist und die Rückseite des Austragskolbens (4, 104) eine dazu als Negativform passende sich stetig in Richtung des Verschlussmittels (10, 110) verjüngende Oberfläche aufweist.

4. Vorrichtung nach einem der vorangehenden Ansprüche, **dadurch gekennzeichnet, dass**
eine Vorderseite des Förderkolbens (3, 103), die dem Austragskolben (4, 104) zugewandt ist, eine Vertiefung mit trichterförmiger Oberfläche (46, 146) aufweist, in deren tiefsten Punkt die Fluidöffnung (8, 108) angeordnet ist, und die Rückseite des Austragskolbens (4, 104) einen vorspringende kegelförmige Oberfläche (44, 144) mit der gleichen Steigung wie die trichterförmige Oberfläche (46, 146) aufweist, wobei an der Spitze der kegelförmigen Oberfläche (44, 144) das Verschlussmittel (10, 110) angeordnet ist.

5. Vorrichtung nach Anspruch 4, **dadurch gekennzeichnet, dass**
die trichterförmige Oberfläche (46, 146) an der Vorderseite des Förderkolbens (3, 103) einen Steigungswinkel von mindestens 45° aufweist, bevorzugt von mindestens 55°, besonders bevorzugt von mindestens 60°, und
die kegelförmige Oberfläche (44, 144) an der Rückseite des Austragskolbens (4, 104) einen dazu passenden Steigungswinkel von mindestens 45° aufweist, bevorzugt von mindestens 55°, besonders bevorzugt von mindestens 60°aufweist.

6. Vorrichtung nach einem der vorangehenden Ansprüche, **dadurch gekennzeichnet, dass**
das Verschlussmittel (10, 110) ein zylindrischer Stab ist, der zumindest mit einer Länge von 10 mm von Rückseite des Austragskolbens (4, 104) absteht, und/oder die Länge des Verschlussmittels (10, 110) so lang ist, dass das Volumen des zweiten Hohlraums (7) höchstens so groß ist wie das Volumen der durch die Fluidöffnung (8, 108) eingeleiteten Monomerflüssigkeit (6), insbesondere der in dem Monomerflüssigkeitsbehälter enthaltenen Monomerflüssigkeit (6), wenn die Spitze des Verschlussmittels (10, 110) auf die Fluidöffnung (8, 108) trifft und diese verschließt.

7. Vorrichtung nach einem der vorangehenden Ansprüche, **dadurch gekennzeichnet, dass**
an einer dem Austragskolben (4, 104) abgewandten Rückseite des Förderkolbens (3, 103) ein Behälter (34, 134) angeordnet ist, in dem ein Monomerflüssigkeitsbehälter (32) enthaltend die Monomerflüssigkeit (6) angeordnet ist, insbesondere eine Ampulle aus Glas oder Kunststoff, wobei der Monomerflüssigkeitsbehälter (32) innerhalb des Behälters (34, 134) zu öffnen ist und wobei der Behälter (34, 134) über die Fluidöffnung (8, 108) mit dem zweiten Hohlraum (7) flüssigkeitsdurchlässig verbunden ist, wobei bevorzugt ein Öffnungsmittel (50) an einer dem Förderkolben (3, 103) gegenüberliegende Seite des Behälters (34, 134) angeordnet ist, mit dem der Monomerflüssigkeitsbehälter (32) innerhalb des Behälters (34, 134) zu öffnen ist, wobei besonders bevorzugt das Öffnungsmittel (50) eine an einer Kappe (54) befestigte Hülse (56) ist, wobei die Kappe (54) auf ein Gewinde des Behälters (34, 134) schraubbar ist und die Kappe (54) hierzu ein Gegengewinde aufweist, so dass beim Aufschrauben der Kappe (54) der Monomerflüssigkeitsbehälter (32), insbesondere die Ampulle, mit der Hülse (56) auf zumindest einen vorstehenden Stift (48) an der Innenseite des Behälters (34, 134) gedrückt wird und dadurch der Monomerflüssigkeitsbehälter (32), insbesondere die Ampulle, aufbrechbar ist.

8. Vorrichtung nach Anspruch 7, **dadurch gekennzeichnet, dass**
der Behälter (34, 134) ein Außengewinde aufweist, das in ein Innengewinde an einem dem Kartuschenkopf (2) gegenüberliegenden Ende der Kartusche (1, 101) schraubbar ist, wobei durch Einschrauben des Behälters (34, 134) in die Kartusche (1, 101) der Förderkolben (3, 103) in Richtung der Austragsöffnung drückbar ist und der Austragskolben (4, 104) mit dem Förderkolben (3, 103) in Richtung der Austragsöffnung drückbar ist, wobei bevorzugt das Innengewinde Teil einer Ringhülse ist, die an dem dem Kartuschenkopf (2) gegenüberliegenden Ende der Kartusche (1, 101) mit der Kartusche (1, 101) verbunden ist.

9. Vorrichtung nach einem der vorangehenden Ansprüche, **dadurch gekennzeichnet, dass**
das Verschlussmittel (10, 110) eine zylindrische Form hat und die Fluidöffnung (8, 108) eine dazu passende Form hat, wobei bevorzugt das Verschlussmittel (10, 110) eine zylindrische Form mit kreisförmiger Grundfläche hat und die Fluidöffnung (8, 108) ein kreisrundes oder zylindrisches Loch ist.

10. Vorrichtung nach einem der vorangehenden Ansprüche, **dadurch gekennzeichnet, dass**
ein Stopfen (12) in der Austragsöffnung angeordnet ist, der die Austragsöffnung für das Zementpulver (5) undurchlässig verschließt, insbesondere für Gase durchlässig verschließt, wobei vorzugsweise der Stopfen (12) in der Austragsöffnung beweglich angeordnet ist, so dass der Stopfen (12) durch einen Druck auf den fertig gemischten Knochenzementteig (62) aus der Austragsöffnung heraus gedrückt werden kann, wobei besonders bevorzugt ein von außen sichtbares Markierungsmittel (16) an dem Stopfen (12) befestigt ist, an dessen Position von außen ablesbar ist, ob der Stopfen (12) in der Austragsöffnung nach außen gedrückt ist.

11. Vorrichtung nach einem der vorangehenden Ansprüche, **dadurch gekennzeichnet, dass**
an dem Kartuschenkopf (2) ein lösbar befestigter Standfuß (18) angeordnet ist, wobei der Standfuß (18) vorzugsweise zumindest teilweise transparent ist, wobei besonders bevorzugt ein dem Kartuschenkopf (2) zugewandter Teil des Standfußes (18) intransparent ist und ein von dem Kartuschenkopf (2) abgewandtes Standfußteil (20) transparent ist.

12. Verfahren zur Herstellung eines Knochenzementteigs (62), insbesondere eines pastenförmigen Polymethylmethacrylat-Knochenzementteigs, wobei der Knochenzementteig (62) aus einem Zementpulver (5) und einer Monomerflüssigkeit (6) mit einer Vorrichtung nach einem der vorangehenden Ansprüche hergestellt wird, **gekennzeichnet durch** die folgenden nacheinander ablaufenden Schritte:
A) Einleiten der Monomerflüssigkeit (6) durch die Fluidöffnung (8, 108) in den zweiten Hohlraum (7),
B) Drücken des Förderkolbens (3, 103) in Richtung des Austragskolbens (4, 104) bis das Verschlussmittel (10, 110) an der Rückseite des Austragskolbens (4, 104) die Fluidöffnung (8, 108) verschließt,
C) Weiteres Drücken des Förderkolbens (3, 103) in Richtung des Austragskolbens (4, 104), wobei die Monomerflüssigkeit (6) aus dem zweiten Hohlraum (7) in das Zementpulver (5) in den ersten Hohlraum (60) gedrückt wird, das Verschlussmittel (10, 110) durch die Fluidöffnung (8, 108) oder tiefer in die Fluidöffnung (8, 108) geschoben wird und die Fluidöffnung (8, 108) verschlossen bleibt,
D) Drücken des Austragskolbens (4, 104) mit dem Förderkolben (3, 103) in Richtung der Austragsöffnung, wobei der in dem ersten Hohlraum (60) gebildete Knochenzementteig (62) durch die Austragsöffnung ausfließt.

13. Verfahren nach Anspruch 12, **dadurch gekennzeichnet, dass**
die Vorrichtung in Schritt A), und vorzugsweise auch in Schritt B) und C), mit dem Kartuschenkopf (2) nach unten aufgestellt oder gehalten wird und Gaseinschlüsse aus dem zweiten Hohlraum (7) durch die Fluidöffnung (8, 108) entweichen.

14. Verfahren nach Anspruch 13 oder 14, **dadurch gekennzeichnet, dass**
in Schritt D) der Förderkolben (3, 103) flächenbündig an dem Austragskolben (4, 104) anliegt und bevorzugt in Schritt C) das Volumen des zweiten Hohlraums (7) vollständig oder bis auf maximal 1% reduziert wird, und/oder
in Schritt D) durch den auf den Knochenzementteig (62) wirkenden Druck ein Stopfen (12) in der Austragsöffnung bewegt oder hervorgedrückt wird, wobei bevorzugt hierauf der Stopfen (12) aus der Austragsöffnung entfernt wird und gegebenenfalls ein Standfuß (18) von dem Kartuschenkopf (2) entfernt wird und besonders bevorzugt danach ein Applikationsrohr an dem Kartuschenkopf (2) der Kartusche (1, 101) befestigt wird.

15. Verfahren nach einem der Ansprüche 12 bis 14, **dadurch gekennzeichnet, dass**
vor Schritt A) ein Monomerflüssigkeitsbehälter (32) enthaltend die Monomerflüssigkeit (6) in einem Behälter (34, 134) geöffnet wird und die Monomerflüssigkeit (6) in dem Behälter (34, 134) freigesetzt wird, wobei der Behälter (34, 134) an einer dem Austragskolben (4, 104) abgewandten Rückseite des Förderkolbens (3, 103) angeordnet ist, und die Monomerflüssigkeit (6) in Schritt A) aus dem Behälter (34, 134) durch die Fluidöffnung (8, 108) in den zweiten Hohlraum (7) fließt, wobei vorzugsweise in Schritt B) und C) der Förderkolben (3, 103) und in Schritt D) der Förderkolben (3, 103) und der Austragskolben (4, 104) durch Eindrücken oder Einschrauben des Behälters (34, 134) in die Kartusche (1, 101) angetrieben werden.

## Claims

1. Device for the production of a bone cement dough (62) from a monomer liquid (6) and a cement powder (5) as starting components of the bone cement dough (62), and for dispensing of the bone cement dough (62), the device comprising a cartridge (1, 101) with a cylindrical internal space;
a cartridge head (2) with a dispensing opening for dispensing the bone cement dough (62), whereby the cartridge head (2) closes the cartridge (1, 101) on a front side of the cartridge (1, 101) except for the dispensing opening;
a conveying plunger (3, 103) that is arranged in the internal space of the cartridge (1, 101) and is supported in the internal space of the cartridge (1, 101) such that it is pushable in the direction of the dispensing opening;
a dispensing plunger (4, 104) that is arranged in the internal space of the cartridge (1, 101) between the dispensing opening and the conveying plunger (3, 103) and that is supported in the internal space of the cartridge (1, 101) such that it is pushable in the direction of the dispensing opening;
a first hollow space (60) that is bordered by the cartridge head (2), by internal walls of the cartridge (1, 101), and by the dispensing plunger (4, 104), whereby the cement powder (5) is arranged in the first hollow space (60);
a second hollow space (7) that is part of the cylindrical internal space of the cartridge (1, 101), whereby the second hollow space (7) is bordered by the dispensing plunger (4, 104) and the conveying plunger (3, 103);
a fluid opening (8, 108) that is arranged in the conveying plunger (3, 103) and through which the monomer liquid (6) is conveyable into the second hollow space (7);
a feedthrough (9, 109) that is arranged in the dispensing plunger (4, 104) and/or in the wall of the cartridge (1, 101) and which connects the first hollow space (60) and the second hollow space (7) in monomer liquid-permeable, but cement powder (5) impermeable manner, and
a closure means (10, 110) that is arranged on a rear side of the dispensing plunger (4, 104) that faces away from the dispensing opening, whereby the closure means (10, 110) projects away from the dispensing plunger (4, 104) in the direction of the conveying plunger (3, 103) and is situated at a distance from the fluid opening (8, 108) in the conveying plunger (3, 103), whereby the fluid opening (8, 108) is closable with the closure means (10, 110) when the conveying plunger (3, 103) is being pushed in the direction of the dispensing opening, and whereby the closure means (10, 110) is appropriately mobile in the fluid opening (8, 108) closed by the closure means (10, 110) such that the fluid opening (8, 108) stays closed, while the conveying plunger (3, 103) is pushable further in the direction of the dispensing plunger (4, 104).

2. Device according to claim 1, **characterised in that**
a front side of the conveying plunger (3, 103) that faces the dispensing plunger (4, 104) and the rear side of the dispensing plunger (4, 104) are shaped with matching surfaces with respect to each other such that the front side of the conveying plunger (3, 103) touches against the rear side of the dispensing plunger (4, 104) such that the surfaces match when the conveying plunger (3, 103) is pushed against the dispensing plunger (4, 104), whereby preferably the volume of the second hollow space (7) thereby is reducible to a maximum of 1% of the volume of the second hollow space (7) in a starting state, whereby the at least one closure means (10, 110) is situated at a distance from the at least one fluid opening (8, 108) in the starting state.

3. Device according to claim 1 or 2, **characterised in that**
a front side of the conveying plunger (3, 103) that faces the dispensing plunger (4, 104) comprises a surface that tapers continuously in the direction of the fluid opening (8, 108), and the rear side of the dispensing plunger (4, 104) comprises a surface that matches it like a negative shape and tapers continuously in the direction of the closure means (10, 110).

4. Device according to any one of the preceding claims, **characterised in that**
a front side of the conveying plunger (3, 103) that faces the dispensing plunger (4, 104) comprises a depression with a funnel-shaped surface (46, 146) whose lowest point has the fluid opening (8, 108) arranged in it, and **in that** the rear side of the dispensing plunger (4, 104) comprises a projecting, cone-shaped surface (44, 144) of the same slope as the funnel-shaped surface (46, 146), whereby the closure means (10, 110) is arranged on the tip of the cone-shaped surface (44, 144).

5. Device according to claim 4, **characterised in that**
the funnel-shaped surface (46, 146) on the front side of the conveying plunger (3, 103) comprises an angle of slope of at least 45°, preferably of at least 55°, particularly preferably of at least 60°, and
the cone-shaped surface (44, 144) on the rear side of the dispensing plunger (4, 104) comprises a matching angle of slope of at least 45°, preferably of at least 55°, particularly preferably of at least 60°.

6. Device according to any one of the preceding claims, **characterised in that**
the closure means (10, 110) is a cylindrical rod that projects away from the rear side of the dispensing plunger (4, 104) by a length of at least 10 mm, and/or
the length of the closure means (10, 110) is appropriate such that the volume of the second hollow space (7) is at most equal to the volume of the monomer liquid (6) conducted through the fluid opening (8, 108), in particular of the monomer liquid (6) contained in the monomer liquid container, when the tip of the closure means (10, 110) encounters and closes the fluid opening (8, 108).

7. Device according to any one of the preceding claims, **characterised in that**
a container (34, 134) is arranged on a rear side of the conveying plunger (3, 103) that faces away from the dispensing plunger (4, 104), with a monomer liquid container (32) containing the monomer liquid (6) being arranged in the container (34, 134), in particular an ampoule made of glass or plastics, whereby the monomer liquid container (32) is openable inside the container (34, 134) and whereby the container (34, 134) is connected to the second hollow space (7) in fluid-permeable manner via the fluid opening (8, 108), whereby an opening means (50) is preferably arranged on a side of the container (34, 134) opposite from the conveying plunger (3, 103), by means of which the monomer liquid container (32) is openable inside the container (34, 134), whereby the opening means (50) is particularly preferred to be a sleeve (56) attached to a cap (54), whereby the cap (54) is screwable onto a thread of the container (34, 134) and the cap (54) comprises a counter-thread for this purpose such that, when the cap (54) is being screwed on, the monomer liquid container (32), in particular the ampoule, is pushed, by the sleeve (56), onto at least one projecting pin (48) on the internal side of the container (34, 134) and thus the monomer liquid container (32), in particular the ampoule, is breakable open.

8. Device according to claim 7, **characterised in that**
the container (34, 134) comprises an external thread that is screwable into an internal thread on an end of the cartridge (1, 101) opposite from the cartridge head (2), whereby the conveying plunger (3, 103) is pushable in the direction of the dispensing opening by screwing the container (34, 134) into the cartridge (1, 101) and the dispensing plunger (4, 104) is pushable by the conveying plunger (3, 103) in the direction of the dispensing opening, whereby the internal thread preferably is part of a ring sleeve that is connected to the cartridge (1, 101) on the end of the cartridge (1, 101) opposite from the cartridge head (2).

9. Device according to any one of the preceding claims, **characterised in that**
the closure means (10, 110) is cylindrical in shape and the fluid opening (8, 108) has a matching shape, whereby the closure means (10, 110) preferably has a cylindrical shape with a circular footprint and the fluid opening (8, 108) is a circular or cylindrical hole.

10. Device according to any one of the preceding claims, **characterised in that**
the dispensing opening has a stopper (12) arranged in it that closes the dispensing opening impermeable to the cement powder (5), in particular closes the dispensing opening permeable to gases, whereby the stopper (12) preferably is arranged in the dispensing opening such as to be mobile such that the stopper (12) is pushable out of the dispensing opening by pressing on the ready-mixed bone cement dough (62), whereby it is particularly preferred to have a marker means (16) that is visible from outside attached to the stopper (12), whose position is readable from outside to indicate whether the stopper (12) is pushed outward in the dispensing opening.

11. Device according to any one of the preceding claims, **characterised in that**
a detachably attached foot (18) is arranged on the cartridge head (2), whereby the foot (18) is preferred to be at least partially transparent, whereby it is particularly preferred for a part of the foot (18) facing the cartridge head (2) to be non-transparent and a part (20) of the foot facing away from the cartridge head (2) to be transparent.

12. Method for the production of a bone cement dough (62), in particular a pasty polymethylmethacrylate bone cement dough, whereby the bone cement dough (62) is produced from a cement powder (5) and a monomer liquid (6) with a device according to any one of the preceding claims, **characterised by** the following steps proceeding in the order given:
A) conducting the monomer liquid (6) through the fluid opening (8, 108) into the second hollow space (7);
B) pushing the conveying plunger (3, 103) in the direction of the dispensing plunger (4, 104) until the closure means (10, 110) on the rear side of the dispensing plunger (4, 104) closes the fluid opening (8, 108);
C) pushing the conveying plunger (3, 103) further in the direction of the dispensing plunger (4, 104), whereby the monomer liquid (6) is pushed from the second hollow space (7) into the cement powder (5) in the first hollow space (60), with the closure means (10, 110) being pushed through the fluid opening (8, 108) or more deeply into the fluid opening (8, 108) and the fluid opening (8, 108) remaining closed;
D) the conveying plunger (3, 103) pushing the dispensing plunger (4, 104) in the direction of the dispensing opening, whereby the bone cement dough (62) produced in the first hollow space (60) flows out through the dispensing opening.

13. Method according to claim 12, **characterised in that**
the device is set up or held with the cartridge head (2) downwards in step A), and preferably in steps B) and C) as well, and gas inclusions escape from the second hollow space (7) through the fluid opening (8, 108).

14. Method according to claim 12 or 13, **characterised in that**
the conveying plunger (3, 103) touches against the dispensing plunger (4, 104) in step D) with matching surfaces and preferably the volume of the second hollow space (7) is reduced completely or down to a maximum of 1% in step C), and/or
the pressure acting on the bone cement dough (62) in step D) moves or pushes forward a stopper (12) in the dispensing opening, whereby the stopper (12) is preferably removed from the dispensing opening subsequently and, if applicable, a foot (18) is removed from the cartridge head (2) and, particularly preferably, then an application tube is attached to the cartridge head (2) of the cartridge (1, 101).

15. Method according to any one of the claims 12 to 14, **characterised in that**
a monomer liquid container (32) containing the monomer liquid (6) is opened in a container (34, 134) before step A) and the monomer liquid (6) is released in the container (34, 134), whereby the container (34, 134) is arranged on a rear side of the conveying plunger (3, 103) that faces away from the dispensing plunger (4, 104), and the monomer liquid (6) flows from the container (34, 134) through the fluid opening (8, 108) into the second hollow space (7) in step A), whereby, preferably, the conveying plunger (3, 103) in steps B) and C) and the conveying plunger (3, 103) and the dispensing plunger (4, 104) in step D) are driven by the container (34, 134) being pushed or screwed into the cartridge (1, 101).

## Revendications

1. Dispositif de fabrication d'une pâte de ciment osseux (62) à partir d'un liquide de monomère (6) et d'une poudre de ciment (5), servant de composants de départ de la pâte de ciment osseux (62), et d'évacuation de la pâte de ciment osseux (62), le dispositif présentant
une cartouche (1, 101) avec un espace intérieur cylindrique,
une tête de cartouche (2) dotée d'un orifice d'évacuation pour l'expulsion de la pâte de ciment osseux (62), où la tête de cartouche (2) obture la cartouche (1, 101) au niveau d'un côté avant de la cartouche (1, 101) à l'exception de l'orifice d'évacuation,
un piston de transport (3, 103), qui est disposé dans l'espace intérieur de la cartouche (1, 101) et qui est logé dans l'espace intérieur de la cartouche (1, 101) en pouvant être pressé en direction de l'orifice d'évacuation,
un piston d'évacuation (4, 104), qui est disposé dans l'espace intérieur de la cartouche (1, 101) entre l'orifice d'évacuation et le piston de transport (3, 103) et qui est logé dans l'espace intérieur de la cartouche (1, 101) en pouvant être pressé en direction de l'orifice d'évacuation
une première cavité (60), qui est délimitée par la tête de cartouche (2), des parois intérieures de la cartouche (1, 101) et par le piston d'évacuation (4, 104), où la poudre de ciment (5) est disposée dans la première cavité (60),
une deuxième cavité (7), qui est une partie de l'espace intérieur cylindrique de la cartouche (1, 101), où la deuxième cavité (7) est délimitée par le piston d'évacuation (4, 104) et le piston de transport (3, 103),
un orifice de fluide (8, 108), qui est disposé dans le piston de transport (3, 103) et par lequel le liquide de monomère (6) peut être introduit dans la deuxième cavité (7),
un passage (9, 109), qui est disposé dans le piston d'évacuation (4, 104) et/ou dans la paroi de la cartouche (1, 101) et qui relie la première cavité (60) et la deuxième cavité (7) l'une avec l'autre en étant perméable pour le liquide de monomère (6) mais ne laissant pas passer la poudre de ciment (5), et
un système d'obturation (10, 110), qui est disposé sur un côté arrière du piston d'évacuation (4, 104) opposé à l'orifice d'évacuation, où le système d'obturation (10, 110) dépasse du piston d'évacuation (4, 104) en direction du piston de transport (3, 103) et est espacé par rapport à l'orifice de fluide (8, 108) dans le piston de transport (3, 103), où l'orifice de fluide (8, 108) peut être fermé avec le système d'obturation (10, 110) lorsque le piston de transport (3, 103) est pressé en direction de l'orifice d'évacuation, et où le système d'obturation (10, 110) est mobile dans l'orifice de fluide (8, 108) fermé avec le système d'obturation (10, 110) de sorte que l'orifice de fluide (8, 108) reste fermé, tandis que le piston de transport (3, 103) peut être davantage pressé en direction du piston d'évacuation (4, 104).

2. Dispositif selon la revendication 1, **caractérisé en ce**
**qu'**un côté avant du piston de transport (3, 103), qui est orienté vers le piston d'évacuation (4, 104), et le côté arrière du piston d'évacuation (4, 104) présentent des formes correspondant l'une à l'autre de manière adjacente en surface, de sorte que le côté avant du piston de transport (3, 103) se plaque à plat de manière adjacente sur le côté arrière du piston d'évacuation (4, 104) lorsque le piston de transport (3, 103) est pressé contre le piston d'évacuation (4, 104), où, de préférence, le volume de la deuxième cavité (7) peut être réduit ainsi à au maximum 1 % du volume de la deuxième cavité (7) dans un état de départ, où l'au moins un système d'obturation (10, 110) est espacé de l'au moins un orifice de fluide (8, 108) dans l'état de départ.

3. Dispositif selon la revendication 1 ou la revendication 2, **caractérisé en ce**
**qu'**un côté avant du piston de transport (3, 103), qui est orienté vers le piston d'évacuation (4, 104), présente une surface s'amenuisant régulièrement en direction de l'orifice de fluide (8, 108) et le côté arrière du piston d'évacuation (4, 104) présente une surface s'amenuisant régulièrement en direction du système d'obturation (10, 110) servant de forme complémentaire à celle-ci.

4. Dispositif selon l'une des revendications précédentes, **caractérisé en ce**
**qu'**un côté avant du piston de transport (3, 103) qui est orienté vers le piston d'évacuation (4, 104)) présente un renfoncement avec une surface en forme d'entonnoir (46, 146), au point le plus bas duquel est disposé l'orifice de fluide (8, 108), et le côté arrière du piston d'évacuation (4, 104) présente une surface en forme de cône (44, 144) saillante avec la même pente que la surface en forme d'entonnoir (46, 146), où le système d'obturation (10, 110) est disposé à la pointe de la surface en forme de cône (44, 144).

5. Dispositif selon la revendication 4, **caractérisé en ce que**
la surface en forme d'entonnoir (46, 146) sur le côté avant du piston de transport (3, 103) présente un angle de la pente d'au moins 45 °, de préférence, d'au moins 55 °, de manière particulièrement préférée d'au moins 60 ° ; et
la surface en forme de cône (44, 144) sur le côté arrière du piston d'évacuation (4, 104) présente un angle de la pente lui correspondant d'au moins 45 °, de préférence, d'au moins 55 °, de manière particulièrement préférée d'au moins 60 °.

6. Dispositif selon l'une des revendications précédentes, **caractérisé en ce que**
le système d'obturation (10, 110) est une tige cylindrique qui dépasse avec une longueur de 10 mm du côté arrière du piston d'évacuation (4, 104), et/ou la longueur du système d'obturation (10, 110) est d'autant plus importante que le volume de la deuxième cavité (7) est au plus aussi grand que le volume du liquide de monomère (6) introduit par l'orifice de fluide (8, 108), notamment du liquide de monomère (6) contenu dans le récipient de liquide de monomère lorsque la pointe du système d'obturation (10, 110) atteint l'orifice de fluide (8, 108) et ferme celui-ci.

7. Dispositif selon l'une des revendications précédentes, **caractérisé en ce**
**qu'**un récipient (34, 134) est disposé sur le côté arrière du piston de transport (3,103) opposé au piston d'évacuation (4, 104), dans lequel est disposé un récipient de liquide de monomère (32) contenant le liquide de monomère (6), notamment une ampoule en verre ou en matière plastique, où le récipient de liquide de monomère (32) peut être ouvert à l'intérieur du récipient (34, 134) et où le récipient (34, 134) est relié avec la deuxième cavité (7) en laissant passer les liquides par le biais de l'orifice de fluide (8, 108), où, de préférence, un moyen d'ouverture (50) est disposé sur un côté du récipient (34, 134) situé en face du piston de transport (3, 103) avec lequel le récipient de liquide de monomère (32) peut être ouvert à l'intérieur du récipient (34, 134), où, de manière particulièrement préférée, le moyen d'ouverture (50) est un manchon (56) fixé sur un capuchon (54), où le capuchon (54) peut être vissé sur un filetage du récipient (34, 134) et le capuchon (54) présente un filetage complémentaire à cette fin de sorte que, lors du vissage du capuchon (54), le récipient de liquide de monomère (32), notamment l'ampoule, est pressé avec le manchon (56) sur au moins une pointe proéminente (48) sur le côté intérieur du récipient (34,134) et ainsi le récipient de liquide de monomère (32), notamment l'ampoule, peut s'ouvrir en se cassant.

8. Dispositif selon la revendication 7, **caractérisé en ce que**
le récipient (34, 134) présente un filetage extérieur qui peut se visser dans un filetage intérieur à une extrémité de la cartouche (1, 101) située en face de la tête de cartouche (2), où, par le vissage du récipient (34, 134) dans la cartouche (1, 101), le piston de transport (3, 103) peut être pressé en direction de l'orifice d'évacuation et le piston d'évacuation (4, 104) peut être pressé en direction de l'orifice d'évacuation avec le piston de transport (3, 103), où, de préférence, le filetage intérieur fait partie d'un manchon annulaire qui est relié avec la cartouche (1, 101) à l'extrémité de la cartouche (1, 101) située en face de la tête de cartouche (2).

9. Dispositif selon l'une des revendications précédentes, **caractérisé en ce que**
le système d'obturation (10, 110) a une forme cylindrique et l'orifice de fluide (8, 108) a une forme lui correspondant, où de préférence, le système d'obturation (10, 110) a une forme cylindrique avec une surface de base en forme de cercle et l'orifice de fluide (8, 108) est un trou arrondi en cercle ou cylindrique.

10. Dispositif selon l'une des revendications précédentes, **caractérisé en ce**
**qu'**un bouchon (12) est disposé dans l'orifice d'évacuation, qui ferme l'orifice d'évacuation pour la poudre de ciment (5) en ne la laissant pas passer, mais laisse en particulier passer les gaz, où, de préférence, le bouchon (12) est disposé mobile dans l'orifice d'évacuation de sorte que le bouchon (12) peut être pressé hors de l'orifice d'évacuation par une pression sur la pâte de ciment osseux (62) mélangée et terminée, où, de manière particulièrement préférée, un moyen de marquage (16) visible de l'extérieur est fixé sur le bouchon (12), à la position duquel on peut voir de l'extérieur si le bouchon (12) est pressé vers l'extérieur dans l'orifice d'évacuation.

11. Dispositif selon l'une des revendications précédentes, **caractérisé en ce**
**qu'**un trépied (18) fixé de manière amovible est disposé sur la tête de cartouche (2), où le trépied (18) est de préférence partiellement transparent, où, de manière particulièrement préférée, une partie du trépied (18) orientée vers la tête de cartouche (2) est non transparente et une partie de trépied (20) opposée à la tête de cartouche (2) est transparente.

12. Procédé de fabrication d'une pâte de ciment osseux (62), notamment d'une pâte de ciment en poly méthyl méthacrylate en forme de pâte, dans lequel la pâte de ciment osseux (62) est fabriquée à partir d'une poudre de ciment (5) et d'un liquide de monomère (6) avec un dispositif selon l'une des revendications précédentes, **caractérisé par** les étapes suivantes se déroulant l'une après l'autre :
A) alimentation du liquide de monomère (6) par l'orifice de fluide (8, 108) dans la deuxième cavité (7),
B) exercice d'une pression sur le piston de transport (3, 103) en direction du piston d'évacuation (4, 104) jusqu'à ce que le système d'obturation (10, 110) ferme l'orifice de fluide (8, 108) sur le côté arrière du piston d'évacuation (4, 104),
C) exercice d'une nouvelle pression sur le piston de transport (3, 103) en direction du piston d'évacuation (4, 104), où le liquide de monomère (6) est pressé hors de la deuxième cavité (7) dans la poudre de ciment (5) dans la première cavité (60), le système d'obturation (10, 110) est poussé à travers l'orifice de fluide (8, 108) ou plus profondément dans l'orifice de fluide (8, 108) et l'orifice de fluide (8, 108) reste fermé,
D) exercice d'une pression sur le piston d'évacuation (4, 104) avec le piston de transport (3, 103) en direction de l'orifice d'évacuation, où la pâte de ciment osseux (62) créée dans la première cavité (60) s'écoule par l'orifice d'évacuation.

13. Procédé selon la revendication 12, **caractérisé en ce que**
le dispositif dans l'étape A), et de préférence, également dans l'étape B), est disposé ou maintenu avec la tête de cartouche (2) vers le bas et que des inclusions de gaz s'échappent à partir de la deuxième cavité (7) à travers l'orifice de fluide (8, 108).

14. Procédé selon la revendication 13 ou la revendication 14, **caractérisé en ce que**
dans l'étape D), le piston de transport (3, 103) est adjacent en surface avec le piston d'évacuation (4, 104) et de préférence dans l'étape C), le volume de la deuxième cavité (7) est réduit totalement ou jusqu'à au maximum 1 %, et/ou
dans l'étape D), par la pression s'exerçant sur la pâte de ciment osseux (62), un bouchon (12) est déplacé ou pressé vers l'avant dans l'orifice d'évacuation, où, de préférence, le bouchon (12) est ensuite enlevé de l'orifice d'évacuation et éventuellement un trépied (18) est retiré de la tête de cartouche (2) et de manière particulièrement préférée, un tube d'application est ensuite fixé sur la tête de cartouche (2) de la cartouche (1, 101).

15. Procédé selon l'une des revendications 12 à 14, **caractérisé en ce**
**qu'**avant l'étape A), un récipient de liquide de monomère (32) contenant le liquide de monomère (6) est ouvert dans un récipient (34, 134), et le liquide de monomère (6) est libéré dans le récipient (34, 134), où le récipient (34, 134) est disposé sur un côté arrière du piston de transport (3, 103) opposé au piston d'évacuation (4, 104), et le liquide de monomère (6) s'écoule dans l'étape A) hors du récipient (34, 134) par l'orifice de fluide (8, 108) dans la deuxième cavité (7), où, de préférence, dans l'étape B) et dans l'étape C), le piston de transport (3, 103), et dans l'étape D), le piston de transport (3, 103) et le piston d'évacuation (4, 104), sont entraînés par l'exercice d'une pression ou par un vissage du récipient (34, 134) dans la cartouche (1, 101).
